# DEMANDE DE BREVET EUROPEEN

(11) **EP 3 498 108 A1**
(43) Date de publication de la demande: **19.06.2019**
(21) Numéro de dépôt: 17306755.4
(22) Date de dépôt: 12.12.2017
(51) Int. Cl.: A23L 33/18, A23C 21/02, A23J 3/34, A61K 38/48, C12P 21/06, C12N 9/48, C12N 9/52, A23L 5/20, A23L 29/00, A23L 33/19, A23L 33/195

(54) **UTILISATION D'UNE COMBINAISON DES EXOPROTÉASES OBTENUS DES MICRO-ORGANISMES EXTREMOPHILES POUR HYDROLYSER DES POLYPEPTIDES**

(71) Demandeur: CENTRE NATIONAL DE LA RECHERCHE SCIENTIFIQUE, 75016 Paris (FR); Commissariat à l'Energie Atomique et aux Energies Alternatives, 75015 Paris (FR); Université Grenoble Alpes, 38400 Saint-Martin-d'Hères (FR)
(72) Inventeur: FRANZETTI, Bruno, 38360 Sassenage (FR); GIRARD, Eric, 26100 ROMANS-SUR-ISERE (FR); APPOLAIRE, Alexandre, 38000 GRENOBLE (FR)
(74) Mandataire: Lavoix

(57) **Abrégé**

L'invention concerne une composition comprenant au moins une première aminopeptidase et au moins une deuxième aminopeptidase, la première aminopeptidase représentant jusqu'à 40 % en poids par rapport au poids total de la composition.

## Description

La présente invention concerne une composition comprenant des aminopeptidases, plus particulièrement une composition qui comprend des aminopeptidases tétraédriques, également appelées « aminopeptidases TET ».

Dans l'industrie agro-alimentaire, des peptides « longs », également qualifiés de « complexes », sont naturellement présents dans les aliments consommés. Ces peptides longs sont généralement issus d'un processus de dégradation insuffisant et sont souvent toxiques, ce qui engendre des intolérances ou allergies aux aliments contenant ces peptides et diminue aussi leur efficacité nutritionnelle. Ces peptides déterminent également les qualités gustatives et la texture de nombreux aliments tels que les pains, les fromages, les salaisons, etc.

L'intolérance au gluten est probablement une des intolérances alimentaires les plus répandues. Elle est causée par une famille de protéines complexes nommée « gliadine » qui entre dans la composition du gluten, ces protéines étant parfois porteuse d'un peptide dit « immunodominant » qui provoque une réaction allergique chez les personnes sensibles ou intolérantes au gluten.

Pour ces raisons, l'industrie agro-alimentaire utilise des enzymes protéolytiques dans de nombreux procédés visant à dégrader le gluten et le rendre moins immunogène. Il s'agit essentiellement d'endoprotéases qui permettent de cliver les protéines, et les peptides longs, en fragments. Ces endoprotéases sont notamment utilisées dans les processus de production en viennoiserie, en fromagerie, en biscuiterie, mais aussi dans la production de jus de fruits ou de bières, et même dans la production d'hydrolysats de protéines destinées à une alimentation spéciale. Ces protéases proviennent généralement de bactéries ou de champignons et leur nature exacte est généralement gardée confidentielle.

Cependant, un inconvénient des protéases actuellement utilisées est qu'elles ne permettent pas la production de peptides suffisamment courts pour que toutes les parties toxiques des peptides alimentaires soient éliminées ou que les peptides puissent être efficacement digérées. Chez les personnes sensibles ou intolérantes au gluten, par exemple, la digestion incomplète du gluten induit la présence du peptide « immunodominant » dans le système digestif et provoque *in fine* les symptômes de la maladie de Coeliaque.

Des aminopeptidases mésophiles ont également déjà été envisagées pour la dégradation de ces peptides alimentaires complexes, mais leur faible activité catalytique et les conditions physicochimiques dans lesquelles elles sont actives limitent leur utilisation à la seule modification du goût des aliments.

L'invention a pour but de remédier à tous ces inconvénients. L'invention concerne donc une composition comprenant au moins une première aminopeptidase et au moins une deuxième aminopeptidase, lesdites première et deuxième aminopeptidases différentes l'une de l'autre, lesdites première et deuxième aminopeptidases étant issues de microorganismes extrêmophiles, lesdites première et deuxième aminopeptidases étant des aminopeptidases de la famille des aminopeptidases tétraédriques ou aminopeptidases TET, ladite première aminopeptidase représentant jusqu'à 40 % en poids par rapport au poids total de la composition, et si lesdites première et deuxième aminopeptidases sont différentes de PhTET2 et PhTET3, alors ladite première aminopeptidase représente jusqu'à 50 % en poids par rapport au poids total de la composition. Les inventeurs ont fait la constatation surprenante selon laquelle l'utilisation d'une composition comprenant au moins deux aminopeptidases TET était capable de dégrader des peptides, notamment en mélange, et exerçant un effet synergique allant au-delà de l'effet additif des activités individuelles de chacune des protéines TET. En effet, au lieu de constater une activité globale de la composition correspondant à la combinaison des activités des différentes aminopeptidases TET présentes dans la composition, les inventeurs ont constaté une activité globale différente et qui peut être modulée par les conditions physicochimiques du milieu réactionnel ou par les interactions/interférences entre les différentes aminopeptidases TET de la composition.

Par « aminopeptidase », on entend une enzyme présentant une activité de clivage des acides aminés par l'extrémité des peptides, polypeptides ou protéines. Par "peptide", on entend dans l'invention une chaîne d'acides aminés comprenant au moins 2 acides aminés. Les peptides peuvent être obtenus soit à partir de la dégradation des protéines, soit à partir de synthèses chimiques. Par "polypeptide", on entend dans l'invention une chaîne d'acides aminés plus grande qu'une chaine d'acides aminés d'un peptide et obtenue à partir de la dégradation de protéines et non d'une synthèse chimique. Les peptides et les polypeptides peuvent avoir une fonction biologique. Toutefois, les peptides et polypeptides ne peuvent pas exercer seuls cette fonction dans le cadre d'un processus cellulaire. Par "protéine", on entend dans l'invention une molécule contenant une chaîne d'acides aminés ayant une fonction biologique et qui se trouve naturellement dans un organisme. Cette fonction biologique fait partie d'un processus naturel de la cellule. La composition comprend donc deux aminopeptidases différentes l'une de l'autre en ce qu'elles présentent chacune une séquence en acides aminés différente l'une par rapport à l'autre. Autrement dit, les aminopeptidases présentent des séquences en acides aminés qui divergent par moins un acide aminé. En d'autres termes, les séquences des deux aminopeptidases divergent l'une de l'autre par un ou plusieurs acides aminés.

Les aminopeptidases tétraédriques ou aminopeptidases TET utilisées dans la composition de l'invention sont issues de microorganismes extrêmophiles et plus particulièrement de microorganismes extrêmophiles marins. Ces aminopeptidases tétraédriques ou aminopeptidases TET appartiennent aux familles de métallo aminopeptidases M42 et M18 selon la classification MEROPS. Autrement dit, les aminopeptidases TET se présentent sous la forme de complexes enzymatiques comprenant 12 sous-unités qui ont la particularité de s'auto-assembler en édifices formant des structures typiques en tétraèdre. Une telle forme participe à la grande stabilité de l'enzyme. Par « métallo aminopeptidases », on signifie que ces aminopeptidases ont toutes en commun la présence d'au moins un ion métallique au sein du site actif.

Par « microorganismes extrêmophiles », on entend des organismes vivants, invisibles à l'oeil nu, qui ne peuvent être observés qu'à l'aide d'un microscope. Ces microorganismes peuvent prendre diverses formes de vie parmi lesquelles figurent les bactéries, les champignons microscopiques, les archéobactéries, les protistes, les algues vertes microscopiques, les animaux du plancton, les planaires, les amibes ou encore les virus. « Extrêmophile » qualifie un organisme qui a pour conditions de vie normales des conditions généralement mortelles pour la plupart des autres organismes. Ces conditions de vie peuvent être une température élevée ou faible, des pressions extrêmes, une salinité, une acidité ou une alcalinité importante du milieu dans lequel vit l'organisme, une présence de radioactivité ou encore une absence de dioxygène ou de lumière.

Ces aminopeptidases TET se distinguent des autres aminopeptidases par le fait qu'elles peuvent être très spécifiques pour certains types d'acides aminés. Par conséquent, il est possible avec les compositions selon l'invention d'associer différentes aminopeptidases TET, afin de dégrader des peptides ou des protéines dits complexes en des peptides suffisamment courts. Dès lors, il est possible de dégrader toutes les parties toxiques pour qu'elles puissent être efficacement digérées. Un tel résultat final est obtenu grâce à une activité aminopeptidase spécifique à la composition utilisée. Cette activité aminopeptidase est établie au regard du contenu en polypeptides du substrat et des parties qu'il est souhaitable de modifier, voire totalement dégrader. Pour ce faire, il est choisi, pour la composition de l'invention, les aminopeptidases TET associées en fonction de leur spécificité pour certains acides aminés et de leur interaction/interférence les unes par rapport aux autres concernant leur activité. De plus, il a été constaté par les inventeurs que certaines aminopeptidases TET présentent, dans certaines conditions, une meilleure activité pour la dégradation de peptides enrichis en un type d'acide aminé particulier que d'autres aminopeptidases TET dont il est connu dans l'état de la technique qu'elles sont spécifiques de ce type d'acide aminé. Autrement dit, les différentes associations d'aminopeptidases TET ne relèvent pas d'une simple addition des activités connues des aminopeptidases TET que l'on associe dans la composition.

Dans la composition selon l'invention, la première aminopeptidase peut représenter jusqu'à 40 % en poids par rapport au poids total de la composition. Par « jusqu'à 40 % par rapport au poids total de la composition », on entend que la première aminopeptidase peut représenter 1 %, 2 %, 3 %, 4 %, 5 %, 6 %, 7 %,8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 %, 35 %, 36 %, 37 %, 38 %, 39 %, ou 40 % par rapport au poids total de la composition. Lorsqu'une composition est constituée uniquement par deux aminopeptidases TET, la deuxième aminopeptidase peut donc représenter au moins 60 % en poids par rapport au poids total de la composition, ce qui signifie 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 % ou 99 %. Alors, la première et la deuxième aminopeptidases TET peuvent être dans des proportions allant de 1 : 99 en poids par rapport au poids total de la composition jusqu'à 40 : 60 en poids par rapport au poids total de la composition, en fonction du poids de la première aminopeptidase par rapport au poids total de la composition.

Toutefois, si la première aminopeptidase et la deuxième aminopeptidase ne sont pas PhTET2 et PhTET3, alors la première aminopeptidase peut représenter jusqu'à 50 % en poids par rapport au poids total de la composition. Par « jusqu'à 50 % par rapport au poids total de la composition», on entend dans l'invention que la première aminopeptidase peut représenter 1 %, 2 %, 3 %, 4 %, 5 %, 6 %, 7 %,8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 %, 35 %, 36 %, 37 %, 38 %, 39 %, 40 %, 41 %, 42 %, 43 %, 44 %, 45 %, 46 %, 47 %, 48 %, 49 % ou 50 % par rapport au poids total de la composition.

Par « lesdites première et deuxième aminopeptidases sont différentes de PhTET2 et PhTET3 », on entend que lesdites première et deuxième aminopeptidases comprennent, consistent essentiellement ou consistent en des molécules d'acides aminés dont les séquences présentent moins de 65 % d'identité avec la séquence PhTET2 (SEQ ID NO : 2) ou la séquence PhTET3 (SEQ ID NO : 3). Par « % d'identité entre deux séquences », on entend que lorsqu'on aligne ces deux séquences en acides aminés pour les comparer, par tout moyen connu de l'homme du métier, ces deux séquences présentent des portions de séquence dont les enchainements en acides aminés sont identiques. L'ensemble de toutes ces portions permet d'établir le pourcentage d'identité entre les deux séquences. « Par moins de 65 % d'identité avec la séquence PhTET2 (SEQ ID NO : 2) ou la séquence PhTET3 (SEQ ID NO : 3) », on entend : 1 %, 2 %, 3 %, 4 %, 5 %, 6 %, 7 %,8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 %, 35 %, 36 %, 37 %, 38 %, 39 %, 40 %, 41 %, 42 %, 43 %, 44 %, 45 %, 46 %, 47 %, 48 %, 49 % 50 %, 51 %, 52 %, 53 %, 54 %, 55 %, 56 %, 57 %, 58 %, 59 %, 60 %, 61 %, 62 %, 63 % ou 64 % d'identité avec la séquence PhTET2 (SEQ ID NO : 2) ou la séquence PhTET3 (SEQ ID NO : 3).

Cela signifie que dans le mode de réalisation où la composition n'est constituée que par les première et la deuxième aminopeptidases TET, la deuxième aminopeptidase TET peut représenter au moins 50 % en poids par rapport au poids total de la composition, ce qui signifie 50 %, 51 %, 52 %, 53 %, 54 %, 55 %, 56 %, 57 %, 58 %, 59 %, 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 % ou 99 %, si la première aminopeptidase et la deuxième aminopeptidase ne sont pas PhTET2 et PhTET3.

Avantageusement, ladite au moins une première aminopeptidase et ladite au moins une deuxième aminopeptidase sont choisies parmi les aminopeptidases du groupe constitué de : PhTET1, PhTET2, PhTET3, PhTET4 et MjTET.

PhTET1 est représentée par la séquence d'acides aminés SEQ ID NO : 1, PhTET2 par la séquence d'acides aminés SEQ ID NO : 2., PhTET3 par la séquence d'acides aminés SEQ ID NO : 3, PhTET4 par la séquence d'acides aminés SEQ ID NO : 4 et MjTET par la séquence d'acides aminés nommée SEQ ID NO : 5.

Autrement dit, les couples de première et deuxième aminopeptidases TET d'une composition selon l'invention peuvent être choisis parmi la liste de couples constituée de : PhTET1-PhTET2, PhTET1-PhTET3, PhTET1-PhTET4, PhTET1-MjTET, PhTET2-PhTET3, PhTET2-PhTET4, PhTET2-MjTET, PhTET3-PhTET4, PhTET3-MjTET, PhTET4-MjTET.

Avantageusement, lesdites aminopeptidases comprennent, consistent essentiellement ou consistent en les molécules d'acides aminés de séquences respectives SEQ ID NO : 1 à SEQ ID NO : 5, ou des protéines présentant une activité aminopeptidase, lesdites protéines comprenant, consistant essentiellement ou consistant en des molécules d'acides aminés dont les séquences présentent au moins 65 % d'identité avec l'une des séquences SEQ ID NO : 1 à SEQ ID NO : 5.

Par « % d'identité avec l'une des séquences SEQ ID NO : 1 à SEQ ID NO : 5 », on entend pour l'invention que les protéines présentent une séquence en acides aminés qui, lorsqu'on l'aligne avec l'une des séquences SEQ ID NO : 1 (séquence de PhTET1), SEQ ID NO : 2 (séquence de PhTET2), SEQ ID NO : 3 (séquence de PhTET3), SEQ ID NO : 4 (séquence de PhTET4) ou SEQ ID NO : 5 (séquence de MjTET) pour comparer les deux séquences, permet d'observer des portions de séquence dont les enchainements en acides aminés sont identiques d'une séquence à l'autre. Par « au moins 65 % d'identité avec l'une des séquences SEQ ID NO : 1 à SEQ ID NO : 5 », cela signifie pour l'invention que le pourcentage d'identité peut être : 65 %, 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 100 % d'identité. Autrement dit, les protéines considérées sont celles présentant une séquence en acide aminé dont le pourcentage d'identité avec l'une des séquences SEQ ID NO : 1 à SEQ ID NO : 5 est l'un de ceux décrits ci-avant et ayant une activité aminopeptidase, c'est-à-dire une activité de clivage d'acides aminés par l'extrémité N-terminal des polymères.

Avantageusement, ladite première aminopeptidase représente jusqu'à 10 % en poids par rapport au poids total de la composition, notamment jusqu'à 5 % en poids par rapport au poids total de la composition.

Ainsi, il est possible de moduler de façon très précise les actions des aminopeptidases de la composition avec l'ajout d'une première aminopeptidase en petites quantités. Dès lors, cet ajout d'une aminopeptidase TET en petites quantités dans la composition permet d'influer, de manière significative, sur la nature des produits obtenus après la mise en contact du contenu en polypeptides d'un substrat avec la composition de l'invention.

Avantageusement, ladite première aminopeptidase représente 50 % en poids par rapport au poids total de la composition, sous réserve que lesdites première et deuxième aminopeptidases sont différentes de PhTET2 et PhTET3.

Autrement dit, dans l'alternative de la composition selon l'invention, où cette dernière n'est constituée que par la première aminopeptidase et la deuxième aminopeptidase, ces deux aminopeptidases TET peuvent être dans des proportions équimolaires, sous réserve que lesdites première et deuxième aminopeptidases soient différentes de PhTET2 et PhTET3.

Avantageusement, la composition de l'invention comprend au moins une troisième aminopeptidase, ladite troisième aminopeptidase étant une aminopeptidase de la famille des aminopeptidases tétraédriques ou aminopeptidases TET. Dans le cas où la composition comporte trois aminopeptidases TET, les triplets peuvent être choisis parmi la liste de triplets constituée de : PhTET1-PhTET2-PhTET3, PhTET1-PhTET2-PhTET4, PhTET1-PhTET2-MjTET, PhTET1-PhTET2-PhTET4, PhTET1-PhTET3-MjTET, PhTET1-PhTET2-PhTET3, PhTET2-PhTET3-PhTET4, PhTET2-PhTET3-MjTET, PhTET2-PhTET4-MjTET. Par ailleurs, dans le cas où la composition comporte quatre aminopeptidases TET, les quadruplets peuvent être choisis parmi la liste de quadruplets constituée de : PhTET1-PhTET2-PhTET3-PhTET4, PhTET1-PhTET2-PhTET3-MjTET, PhTET2-PhTET3-PhTET4-MjTET. Par ailleurs, dans le cas où la composition comporterait cinq aminopeptidases, le quintuplet peut être PhTET1-PhTET2-PhTET3-PhTET4-MjTET.

Ainsi, il est possible d'ajouter l'action d'une troisième aminopeptidase TET, différente de la première aminopeptidase TET et de la deuxième aminopeptidase TET, qui permet d'améliorer le champ d'action de la composition et ainsi de modifier encore mieux les peptides, polypeptides et protéines alimentaires ciblés. Dès lors, la première aminopeptidase TET peut représenter jusqu'à 40 % en poids par rapport au poids total de la composition, les au moins 60 % en poids par rapport au poids total de la composition restants pouvant être répartis entre la deuxième aminopeptidase TET et la troisième aminopeptidase TET.

Toutefois, lorsque les première et deuxième aminopeptidases sont différentes de PhTET2 et PhTET3, la composition peut être dans des proportions telles que la première aminopeptidase TET représente jusqu'à 50 % en poids par rapport au poids total de la composition, les au moins 50 % en poids par rapport au poids total de la composition restants pouvant être répartis entre la deuxième aminopeptidase TET et la troisième aminopeptidase TET.

A titre d'exemples, il est donc des modes de réalisation selon lesquels la composition est constituée par une première aminopeptidase TET, une deuxième aminopeptidase TET et une troisième aminopeptidase TET, et selon lesquels les proportions en poids par rapport au poids total de la composition des première, deuxièmes et troisièmes aminopeptidases sont les suivantes : 50/25/25, 40/30/30, 40/40/20, 10/10/80 ou encore 10/20/70.

Avantageusement, lesdites première, deuxième et troisième aminopeptidases sont dans des proportions équimolaires ou sensiblement équimolaires.

Par « proportions équimolaire ou sensiblement équimolaires », on entend que les quantités des première, deuxième et troisième aminopeptidases sont les mêmes ou alors sensiblement les mêmes, c'est à dire qu'elles sont très proches les unes par rapport aux autres. Une telle précision permet de définir précisément l'activité aminopeptidase d'une composition et ainsi de mieux s'adapter au peptide alimentaire à dégrader.

Avantageusement, la composition comprend en outre une endopeptidase, notamment la thermolysine, en particulier la thermolysine de séquence SEQ ID NO : 6. La thermolysine est une endopeptidase appartenant à la famille des métalloprotéinases. La thermolysine est le membre le plus stable d'une famille de métalloprotéinases produites par diverses espèces de Bacillus. Contrairement à de nombreuses protéines qui subissent des changements conformationnels lors du chauffage et de la dénaturation, la thermolysine ne subit aucun changement conformationnel majeur jusqu'à au moins 70°C. La thermolysine reste donc stable et active aux températures où les protéines TET sont les plus actives.

Par « endopeptidase », on entend une enzyme capable de couper les liaisons entre des acides aminés non-terminaux d'un peptide, d'un polypeptide ou d'une protéine.

Ainsi, la composition présente une activité aminopeptidase plus large grâce à la possibilité de cliver des acides aminés non-terminaux.

L'invention concerne également une utilisation d'une composition selon l'invention pour la modification de tout ou partie du contenu en polypeptides d'un substrat comprenant des peptides, des polypeptides et/ou des protéines.

Par « modification de tout ou partie du contenu en polypeptides d'un substrat », on entend au moins une modification d'une partie du contenu en polypeptides d'un substrat. Autrement dit, le contenu en polypeptides du substrat est différent du contenu en polypeptides du produit obtenu après la mise en contact avec la composition selon l'invention. Par exemple, dans le cas d'un contenu en polypeptides comprenant trois protéines, la modification de tout le contenu correspond à la dégradation intégrale des trois protéines. A l'inverse, la modification d'une partie de ce contenu peut correspondre à la dégradation intégrale d'une protéine sur les trois ou à la modification partielle d'une protéine, de deux protéines sur les trois, voire même des trois protéines du substrat. L'homme du métier est en mesure de déterminer si un contenu en protéine est intégralement ou partiellement modifié.

Dans l'invention, il n'est pas fait de distinction entre les expressions « modification de tout ou partie du contenu en polypeptides d'un substrat » et « dégradation de tout ou partie du contenu en polypeptides d'un substrat ». Ces expressions peuvent se substituer l'une à l'autre sans conséquence quant à l'objet de l'invention. En effet, une modification d'un contenu en polypeptides est le résultat d'une dégradation d'au moins une des constituants (peptide, polypeptide ou protéine) de ce contenu.

Ainsi, la composition selon l'invention est utilisée pour modifier, tout ou partie du contenu en polypeptides d'un substrat qui comprend des peptides, des polypeptides et/ou des protéines. Ces peptides, des polypeptides et/ou des protéines subissent l'action des aminopeptidases TET formant tout ou partie de la composition et sont ainsi modifiés en peptides courts, ce qui permet l'élimination de leur partie toxique et une meilleure digestion du contenu peptidique du produit obtenu après mise en contact du contenu en polypeptides du substrat avec la composition.

Avantageusement, le substrat comprend au moins des peptides, polypeptides et/ou des protéines du gluten et/ou du lactosérum.

Ainsi, il est possible avec l'utilisation de la composition selon l'invention de modifier les peptides, polypeptides et/ou des protéines du gluten ou du lactosérum, ces deux ensembles protéiques comprenant des peptides, polypeptides et/ou des protéines qui sont la cause d'intolérances et d'allergies alimentaires pour les consommateurs finaux. Comme évoqué précédemment, le gluten est principalement constitué de deux familles de protéines : les gliadines et les gluténines. Ces protéines sont insolubles dans l'eau et donnent à la pâte, obtenue après réhydratation de la farine, des propriétés viscoélastiques exploitées dans le domaine agroalimentaire pour donner une certaine structure aux produits. Le lactosérum contient la plus grande partie de l'eau du lait. Il est constitué de 94 % d'eau, de 4 à 5 % de lactose, de protéines solubles (9 % de matière sèche), et de sels minéraux. Les protéines du lactosérum possèdent un véritable intérêt nutritionnel en raison de leur composition élevée en acides aminés essentiels. Les plus importantes sont la β-lactoglobuline (β-LG), α-lactalbumine (α-LA), les immunoglobulines bovines (IgG), l'albumine sérique bovine (BSA) et la lactoferrine bovine (LF).

Avantageusement, le substrat comprend au moins une des protéines suivantes : gliadine, β-lactoglobuline, l'a-lactalbumine, immunoglobulines, albumine de sérum et lactoferrine.

Comme évoquée précédemment, l'utilisation de la composition selon l'invention permet la modification de ces protéines qui sont la cause d'intolérances et d'allergies alimentaires pour les consommateurs finaux.

Avantageusement, lesdites aminopeptidases formant tout ou partie de la composition sont utilisées de manière simultanée, séparée ou étalée dans le temps.

Cette possibilité d'utiliser ces aminopeptidases de manière simultanée, séparée ou étalée dans le temps, permet l'adaptation de l'activité globale de la composition au contenu en polypeptides du substrat sur lequel la composition est utilisée. En effet, il est possible de recourir aux différentes activités aminopeptidases de la composition, fonctions entre autres des aminopeptidases, de leur proportion et de leur interaction les unes avec les autres en termes d'activité, afin d'adapter l'action de modification du contenu en polypeptides du substrat, dans le temps. Les aminopeptidases formant la composition peuvent être utilisées simultanément. A ce titre, elles peuvent, par exemple, être ajoutées en même temps dans un milieu comprenant le substrat à modifier. Les aminopeptidases peuvent également être utilisées de manière séparée. Par exemple, dans le cas d'un contenant de grande taille, une partie des aminopeptidases peut être ajoutée à une extrémité du contenant tandis qu'une autre partie est ajoutée à une autre extrémité du contenant. Une telle solution peut être envisagée dans le but d'obtenir plus rapidement une répartition homogène au sein du contenant des aminopeptidases ajoutées. Cette solution peut être également être envisagée lorsqu'une aminopeptidase introduite à une extrémité du contenant nécessite une certaine période d'adaptation au sein du milieu avant d'être opérationnelle pour une mise en contact avec d'autres aminopeptidases ajoutées à une autre extrémité du contenant. Les aminopeptidases peuvent également être utilisées de manière étalée dans le temps. Ainsi, il est possible d'ajouter une aminopeptidase, pendant un certain temps, afin de modifier le contenu polypeptidique du substrat. Dès lors, il est possible d'ajouter une ou plusieurs autres aminopeptidases de façon à modifier tout ou partie du contenu polypeptidique résultant de la modification par la première aminopeptidase, en tenant compte des interactions/interférences de la première aminopeptidase déjà présente dans le milieu réactionnel sur l'activité de cette nouvelle aminopeptidase ajoutée.

Dans un aspect avantageux de l'invention, les protéines TET peuvent être fixées sur un support, notamment une colonne, de la silice ou encore des billes magnétiques, ou tout autre support approprié pour la fixation des protéines.

L'invention concerne également une méthode de modification de tout ou partie du contenu en polypeptides d'un substrat comprenant des peptides, polypeptides et/ou des protéines, ladite méthode comprenant une étape de mise en contact :
- dudit substrat avec
- une composition selon l'invention susmentionnée,
ladite au moins une première aminopeptidase et ladite au moins deuxième aminopeptidase pouvant être activées à une température supérieure à 80°C, et comprenant éventuellement, préalablement à ladite étape de mise en contact, une étape de dénaturation des polypeptides dudit substrat.

Par « étape de mise en contact », on entend une étape dans laquelle le contenu en polypeptides du substrat et les aminopeptidases TET de la composition peuvent interagir les uns avec les autres, en vue d'une modification du contenu en polypeptides du substrat.

Par « une première aminopeptidase et ladite au moins deuxième aminopeptidase pouvant être activées à une température supérieure à 80°C », on entend que les aminopeptidases peuvent passer d'un stade dans lequel elles ne clivent pas les acides aminés des peptides, polypeptides et/ou des protéines du substrat à un stade où elles clivent ces acides aminés.

Les polypeptides du substrat peuvent préalablement subir une étape de dénaturation, par exemple une dénaturation au propanol, ce qui a pour effet de favoriser leur modification par les aminopeptidases de la composition. Ces polypeptides sont ceux qui possèdent naturellement une structure tertiaire. Dès lors, les solvants organiques comme le propanol provoque une destruction des liaisons non covalentes, par exemple les liaisons hydrogènes internes des polypeptides. Or, de telles liaisons stabilisent la structure tertiaire des polypeptides. Une telle destruction provoque, par conséquent, une déstabilisation, un dépliement, voire une dénaturation des polypeptides.

L'invention concerne également un composé alimentaire susceptible d'être obtenu par la méthode de l'invention.

L'invention concerne également un composé alimentaire comprenant au moins une des protéines suivantes sous forme modifiée : gliadine, β-lactoglobuline, l'a-lactalbumine, immunoglobulines, albumine de sérum et lactoferrine, ledit composé alimentaire comprenant en outre une composition selon l'invention.

L'invention sera mieux comprise à la lumière des figures annexées, qui sont fournies à titre d'exemples et ne présentent aucun caractère limitatif.

### BREVE DESCRIPTION DES FIGURES :

Toutes les figures qui suivent représentent des chromatogrammes illustrant une absorbance (exprimée en mAu) en fonction d'un temps d'élution (exprimé en seconde).

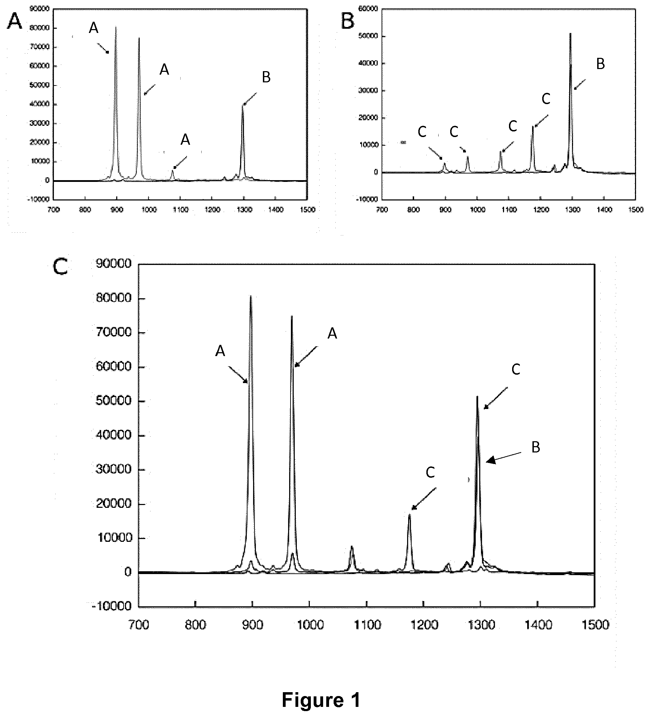

- les **figures 1A, 1B et 1C** sont des superpositions de chromatogrammes résultant de l'analyse en chromatographie phase inverse (RP-HPLC) de l'hydrolyse du peptide synthétique 2 incubé pendant 15 min avec PhTET3 (figure 1A et courbes A) et PhTET2 (figure 1B et courbes C). L'échantillon contrôle, soit le peptide incubé sans peptidase, est représenté à la courbe B. La figure 1C représente la superposition des 3 chromatogrammes ;

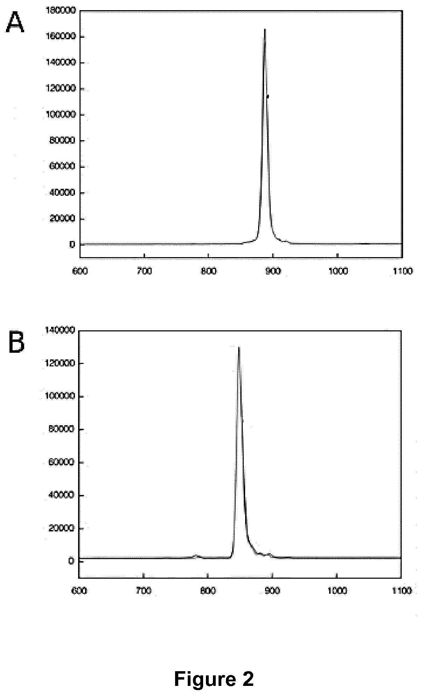

- les **figures 2A et 2B** sont des superpositions des chromatogrammes résultants de l'analyse en chromatographie phase inverse (RP-HPLC) de l'hydrolyse du peptide synthétique 4 incubé pendant 15 min avec PhTET4 (figure 2A) et peptide synthétique 1 incubé pendant 15 min avec PhTET1 (figure 2B) ;

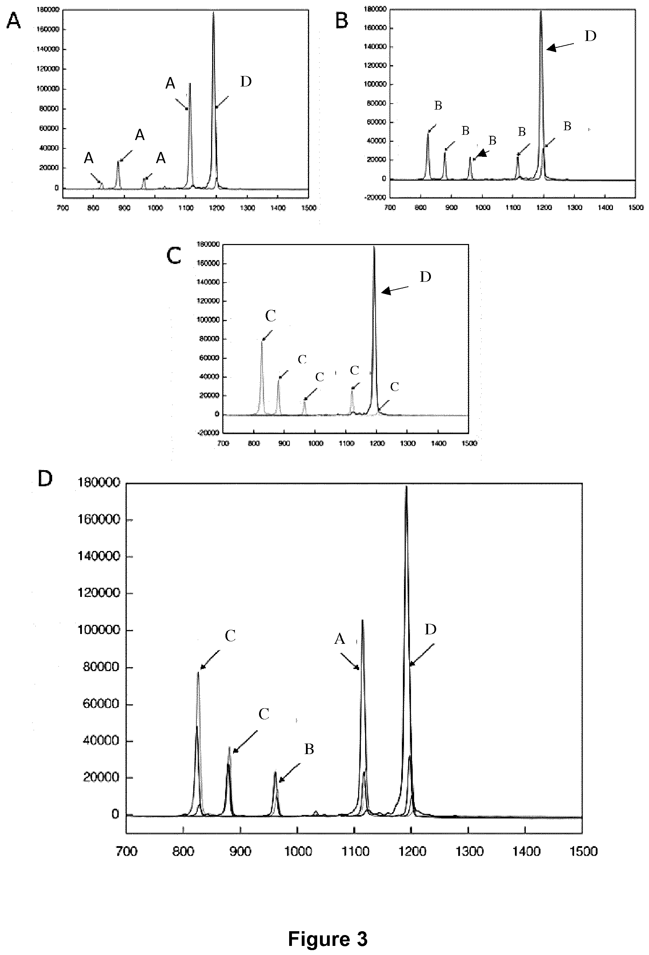

- les **figures 3A, 3B, 3C et 3D** sont des superpositions de chromatogrammes résultant de l'analyse en chromatographie phase inverse (RP-HPLC) de l'hydrolyse du peptide synthétique 5 incubé pendant 15 min avec PhTET3 (figure 3A et courbes A), avec MjTET (figure 3B et courbes B) ou avec un mélange de 10 % de PhTET3 / 90 % de MjTET (figure 3C et courbes C). L'échantillon contrôle, soit le peptide incubé sans peptidase, est représenté aux courbes D. La figure 3D représente la superposition des 4 chromatogrammes ;

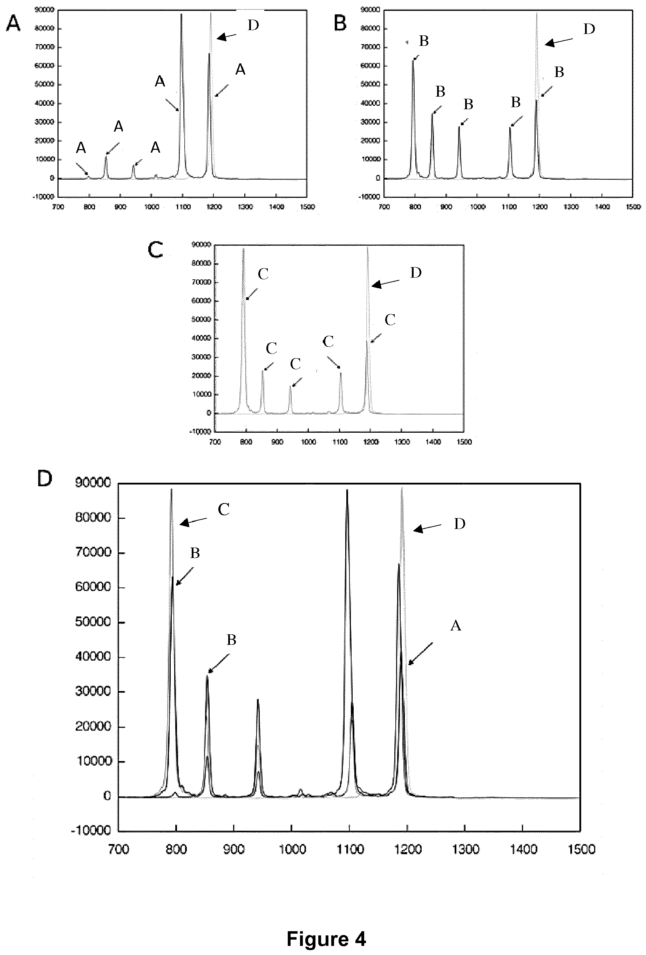

- les **figures 4A, 4B, 4C et 4D** sont des superpositions de chromatogrammes résultant de l'analyse en chromatographie phase inverse (RP-HPLC) de l'hydrolyse du peptide synthétique 5 incubé pendant 15 min avec PhTET3 (figure 4A et courbes A), avec MjTET (figure 4B et courbes B) ou avec un mélange de 5 % de PhTET3 / 95 % de MjTET (figure 4C et courbes C). L'échantillon contrôle, soit le peptide incubé sans peptidase, est représenté aux courbes D. La figure 4D représente la superposition des 4 chromatogrammes ;

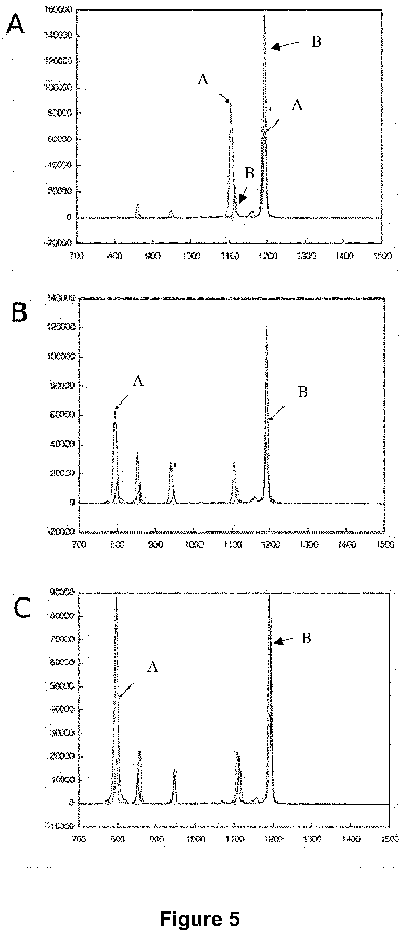

- les **figures 5A, 5B et 5C** sont des superpositions de chromatogrammes résultant de l'analyse en chromatographie phase inverse (RP-HPLC) de l'hydrolyse du peptide synthétique 5 incubé pendant 15 min à des températures différentes, à 40 °C (courbes A) ou 60 °C (courbes B) avec PhTET3 (figure 5A) avec MjTET (figure 5B) ou avec un mélange de 5 % de PhTET3 / 95 % de MjTET (figure 5C). L'échantillon contrôle, soit le peptide incubé sans peptidase, n'est pas visible sur les figures 5A, 5B et 5C ;

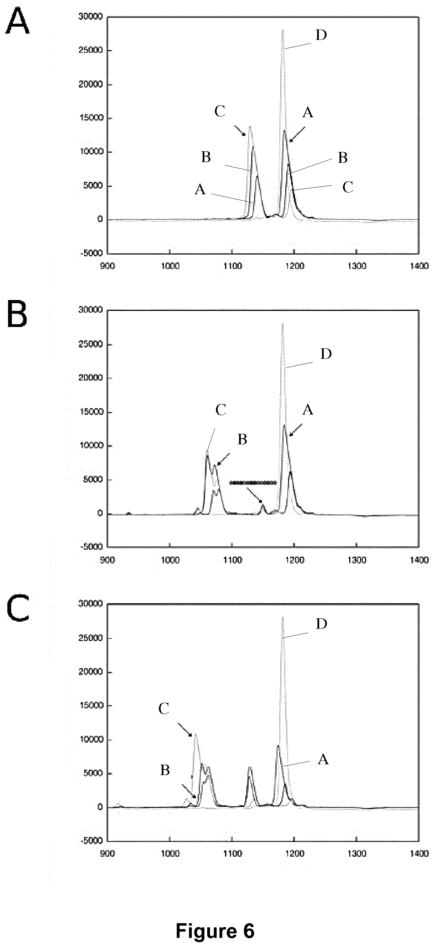

- les **figures 6A, 6B et 6C** sont des superpositions de chromatogrammes résultant de l'analyse en chromatographie phase inverse (RP-HPLC) de la cinétique de l'hydrolyse du peptide synthétique 7 incubé pendant 5, 15 ou 30 min avec PhTET4 (figure 6A), avec MjTET (figure 6B) ou avec un mélange de 50 % de PhTET4 / 50 % de MjTET (figure 6C). Courbes A : 5 min ; courbes B : 15 min ; courbes C : 30 min. L'échantillon contrôle, soit le peptide incubé sans peptidase, est représenté en courbes D ;

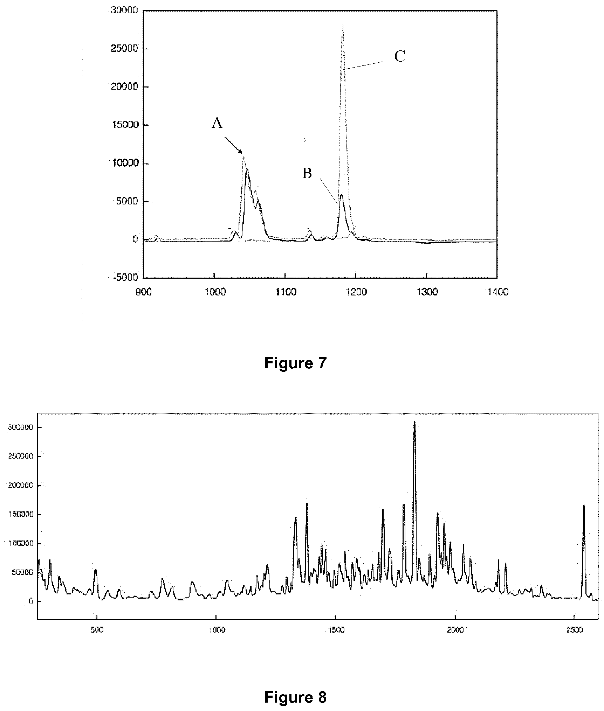

- la **figure 7** représente une superposition de chromatogrammes résultant de l'analyse en chromatographie phase inverse (RP-HPLC) de la cinétique de l'hydrolyse du peptide synthétique 7 incubé pendant 30 min avec MjTET (courbe A) ou avec un mélange de 50 % de PhTET4 / 50 % de MjTET (courbe B). L'échantillon contrôle, soit le peptide incubé sans peptidase, est représenté à la courbe C ;
- la **figure 8** représente un chromatogramme résultant de l'analyse de l'hydrolysat de protéines du petit lait en chromatographie phase inverse (RP-HPLC) ;

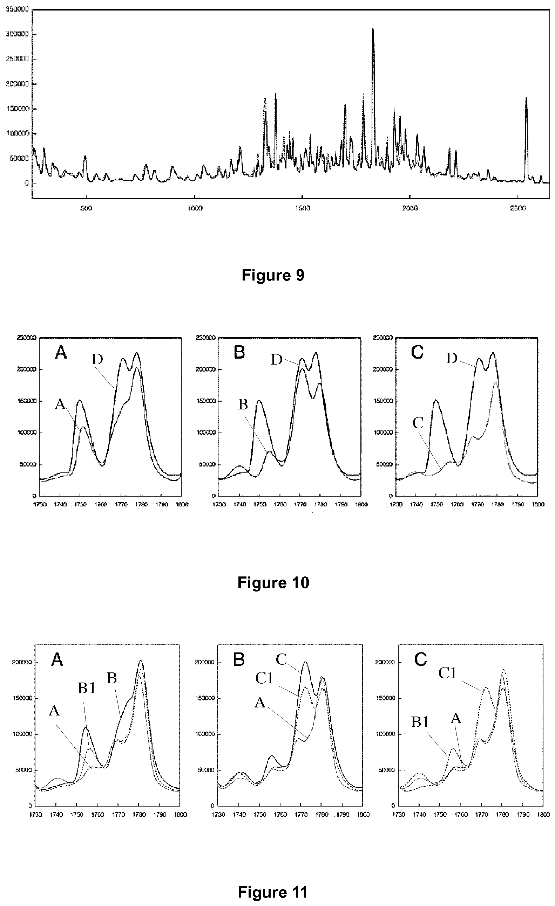

- la **figure 9** représente une superposition des chromatogrammes résultants de l'analyse de l'hydrolysat de protéines du petit lait à pH = 6,2 (trait plein) et celui à pH = 9,5 (trait pointillé) en chromatographie phase inverse (RP-HPLC). On observe peu de différences entre les hydrolysats ;
- la **figure 10** représente une superposition des chromatogrammes résultants de l'analyse de l'hydrolysat de protéines du petit lait à pH = 6,2 seul (courbe D) et après différentes incubations avec différentes TET. (Seule une partie du chromatogramme est représentée.) La figure 10A représente l'incubation avec PhTET2 seule (courbe A). La figure 10B représente l'incubation avec PhTET3 seule (courbe B). La figure 10C représente l'incubation avec PhTET2 et PhTET3 en quantité équimolaire (courbe C) ;
- la **figure 11** représente une superposition des chromatogrammes résultants de l'analyse de l'hydrolysat de protéines du petit lait après incubation avec PhTET2 et PhTET3 en quantité équimolaire (courbes A) comparé à différentes compositions d'aminopeptidases TET. (Seule une partie du chromatogramme est représentée.) La figure 11A représente l'incubation avec PhTET2 seule (courbe B) et un mélange de 90 % de PhTET2 et 10 % de PhTET3 (courbe B1). La figure 11B représente l'incubation avec PhTET3 seule (courbe C) et une composition de 10 % de PhTET2 et 90 % de PhTET3 (courbe C1). La figure 11C représente l'incubation avec une composition de 90 % de PhTET2 et 10 % de PhTET3 (courbe B1) et avec une composition de 10% de PhTET2 et 90 % de PhTET3 (courbe C1) ;

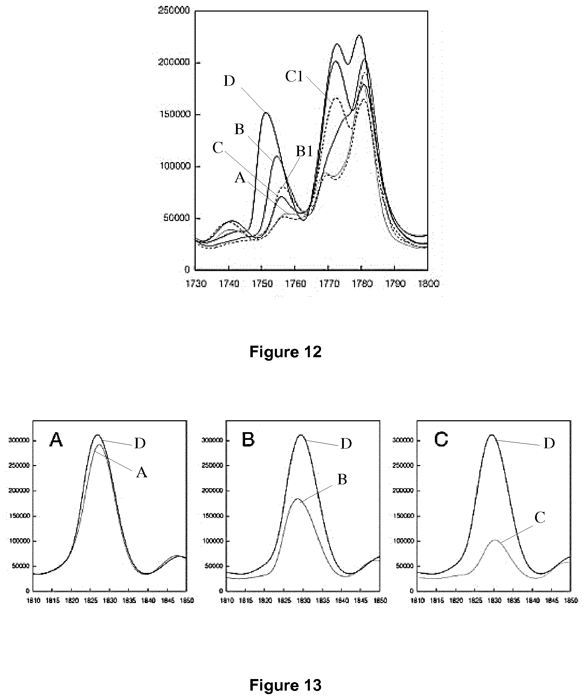

- la **figure 12** représente une superposition des chromatogrammes des figures 11A, 11B et 11C et du chromatogramme résultants de l'analyse de l'hydrolysat de protéines du petit lait à pH = 6,2 seul (courbe D). Seule une partie du chromatogramme est représentée ;
- la figure 13 représente une superposition des chromatogrammes résultants de l'analyse de l'hydrolysat de protéines du petit lait à pH = 9,5 seul (courbe D) et après incubation avec différentes aminopeptidases TET. (Seule une partie du chromatogramme est représentée.).
- La **figure 13A** représente l'incubation avec PhTET4 seule (courbe A). La figure 13B représente l'incubation avec MjTET seule (courbe B). La figure 13C représente l'incubation avec la composition de PhTET4 et MjTET en quantité équimolaire (courbe C) ;

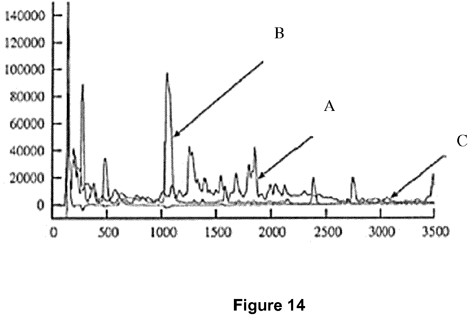

- la **figure 14** représente une superposition des chromatogrammes obtenus en HPLC en phase inverse (colonne ZORBAX SB-300 C18). Courbe A : échantillon de petit lait incubé en présence de la thermolysine. Courbe B : échantillon de petit lait incubé en présence de la thermolysine et des aminopeptidases TET. Courbe C : échantillon de petit lait incubé seul. La colonne utilisé ici permet d'analyser des petits peptides, les protéines « entières » du petit ne sont donc pas visibles.

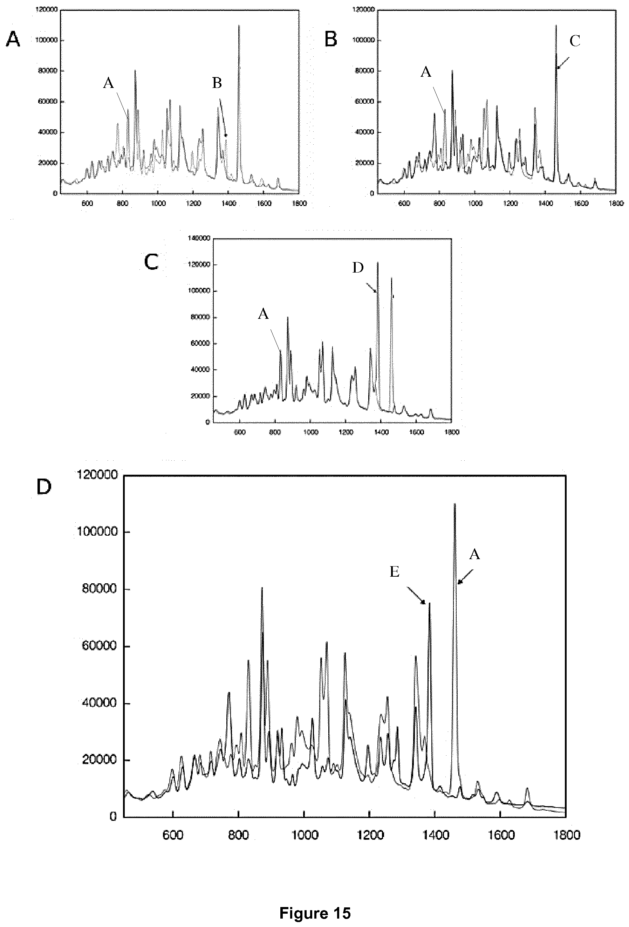

- la **figure 15** représente une superposition des chromatogrammes résultant de l'analyse en chromatographie phase inverse (RP-HPLC) de l'hydrolyse du peptide synthétique 7 incorporé à un hydrolysat de caséine incubé avec PhTET3 (figure 15A), avec MjTET (figure 15B), avec PhTET4 (figure 15C) ou avec une composition de 33 % de PhTET3 / 33 % de PhTET4 / 33 % de MjTET (figure 15D). Courbe A : contrôle (peptide 7 incorporé à l'hydrolysat de caséine sans enzyme) ; courbe B : PhTET3 ; courbe C : MjTET ; courbe D : PhTET4 ; courbe E : mélange de 33 % de PhTET3 / 33 % de PhTET4 / 33 % de MjTET.

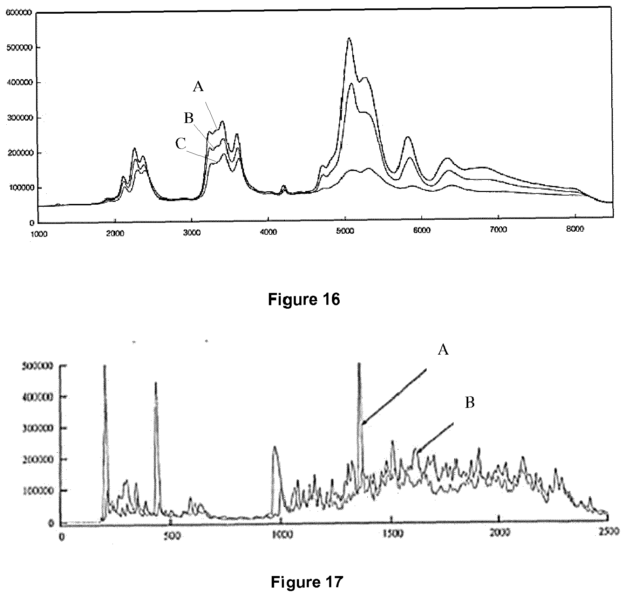

- la **figure 16** représente une superposition des chromatogrammes résultants de l'analyse d'échantillons de gluten : contrôle non incubé (courbe A), incubé sans enzymes (courbe B), incubé avec les enzymes PhTET1, PhTET2 et PhTET3 en quantité équimolaire (trait C). Après 2h d'incubation, on note la diminution de plusieurs pics d'absorbance traduisant la dégradation significative de plusieurs protéines du gluten ;
- la **figure 17** représente une superposition des chromatogrammes résultant de l'analyse en chromatographie phase inverse (RP-HPLC) de l'hydrolyse d'échantillons de gluten. Courbe A : échantillon de gluten total incubé en présence de la thermolysine. Courbe B : échantillon de gluten total incubé en présence de la thermolysine et des aminopeptidases TET PhTET1, PhTET2 et PhTET3.

### EXEMPLE :

### Matériel et méthodes

### Test d'activité des aminopeptidases TET sur des peptides synthétiques

Afin de tester l'activité des aminopeptidases TET sur des peptides synthétiques, différents mélanges d'aminopeptidases TET à une concentration totale de 50 µg / mL sont incubés avec différents peptides à une concentration finale de 0,5 mM dans un volume finale de 100 µL. Un standard interne est ajouté aux expériences en fin de réaction (non visible sur les chromatogrammes présentés), un échantillon de tryptophane à 50 µM. Ce standard interne (ou étalon interne) est utilisé afin d'augmenter la précision des calculs de quantité de produit dans le milieu. Autrement dit, il est connu précisément la quantité de standard injecté sur la colonne et il est ainsi possible de normaliser le signal réponse obtenu. Le standard est un composé qui ne réagit pas dans l'expérience et dont la réponse au signal est très proche des produits mesurés. Dans le cas présent, le standard interne choisi est le tryptophane. Le standard interne est ajouté à la concentration indiquée dans l'échantillon avant son analyse en RP-HPLC. Les tests d'activité sont réalisés à pH = 7,5 dans un tampon PIPES 50 mM, KCl 150 mM, sauf ceux dans lesquels est présente PhTET4 qui sont réalisés à pH = 9,5 dans un tampon CHES 50 mM, KCl 150 mM. Le milieu réactionnel est ensuite incubé pendant différents temps aux températures souhaitées (40 °C ou 60 °C) sous agitation (500 rpm). Les tubes sont ensuite placés dans la glace afin d'arrêter la réaction d'hydrolyse. Ensuite, 80 µL du milieu réactionnel sont ajoutés à 320 µL d'une solution composée de 2 % d'acétonitrile (ACN) et de 0,1 % d'acide trifluoroacétique (TFA). Les échantillons sont alors centrifugés à 10 000 g pendant 10 min avant d'être transférés dans des fioles avant leur injection sur une colonne RP-HPLC pour analyse. En plus de leur analyse en RP-HPLC, les milieux réactionnels de ces tests d'activités ont également été analysés par spectrométrie de masse afin d'identifier précisément la taille des produits d'hydrolyse observés. Ceci a permis d'identifier les différents pics observés sur les chromatogrammes et de suivre de façon optimale les processus d'hydrolyses.

### Préparation d'un hydrolysat de protéines du petit lait de vache

Le petit lait représente la fraction liquide obtenu après la coagulation du lait, c'est un sous-produit obtenu notamment dans l'industrie fromagère. Il contient environ 10 % de protéine qui se répartissent en 5 familles principales : la β-lactoglobuline (50 %), l'a-lactalbumine (20 %), les immunoglobulines (10 %), l'albumine de sérum bovin (10 %) et la lactoferrine (2,8 %). Dans le cas présent, ces différentes protéines sont hydrolysées et les peptides résultants sont utilisés comme substrat modèle. Une solution de petit lait de vache est incubée en présence de thermolysine (Sigma®) à une concentration finale de 100 µg / mL pendant 2 h à 60 °C sous agitation (500 rpm). Après hydrolyse, la solution est incubée pendant 15 min à 95 °C afin d'inactiver la thermolysine. L'hydrolysat de protéines de petit lait est ensuite aliquoté et conservé à -20 °C jusqu'à utilisation.

### Test d'activité des aminopeptidases TET sur un hvdrolvsat de protéines de petit lait

Afin de tester l'activité d'hydrolyse des aminopeptidases TET sur les peptides présents dans l'hydrolysat de protéines de petit lait, différents mélanges de protéines TET à une concentration totale de 50 µg / mL sont incubés avec l'hydrolysat dans un volume final de 100 µL. Aucun cofacteur n'est ajouté à la réaction. Les tests d'activités de PhTET2 et PhTET3 ont été réalisées sur un hydrolysat de protéines de petit lait à son pH natif soit 6,2. La série de test conduite avec PhTET4 et MjTET a été réalisée à pH = 9,5. Le milieu réactionnel est ensuite incubé pendant 2 h à 60 °C sous agitation (500 rpm). Les tubes sont ensuite placés dans la glace afin d'arrêter la réaction d'hydrolyse. Ensuite, 80 µL du milieu réactionnel sont ajoutés à 320 µL d'une solution composée de 2 % d'acétonitrile (ACN) et de 0,1 % d'acide trifluoroacétique (TFA). Les échantillons sont alors centrifugés à 10 000 g pendant 10 min avant d'être transférés dans des fioles avant leur injection sur une colonne RP-HPLC pour analyse.

### Analyse HPLC en phase inverse (RP-HPLC)

100 µL de chaque échantillon est injecté soit sur une colonne µRPC C2/C18 (4,6 mm x 100 mm) (GE heaIthcare®) pour les études sur les peptides, soit sur une colonne ZORBAX SB-300 C8 (4,6 mm x 150 mm) (Agilent®) branché sur un système HPLC Perkin Elmer® pour les études sur le petit lait. La phase A est composé de TFA à 0.1% et d'ACN à 2 % dans de l'eau, la phase B contient quant à elle du TFA à 0.1% et de l'ACN à 80% dans de l'eau. Les protéines adsorbées sont alors éluées à 1 mL / min avec un gradient linéaire 0-50 % de phase B et sont détectées en mesurant leurs absorbances à 280 nm pour les études sur les peptides ou 214 nm pour les autres études. Les pics de protéines sont identifiés et analysés grâce au logiciel TotalChrom version 6.3.1 (Perkin Elmer®).

### Test d'activité des aminopeptidases TET contre les protéines du gluten

Une suspension de protéines de gluten (Sigma®) à 5 % est préparée à l'aide d'un agitateur métallique dans une solution permettant leur solubilisation (150 mM NaCl, 20 mM Tris-HCI, 50 % de propanol, pH = 7,5). 95 µL de cette solution contenant le substrat sont transférés dans des tubes Eppendorf de 0,5 mL. Les tubes sont placés dans un agitateur orbital muni d'un thermostat, ils sont alors incubés à 60 °C sous agitation (500 rpm) pendant 10 min. Une solution contenant le mélange des 3 enzymes PhTET1, PhTET2 et PhTET3 en quantité équimolaire pour une concentration finale de 250 µg / mL est préparée et incubée dans les mêmes conditions. La réaction est démarrée en ajoutant 5 µL du mélange d'enzymes au milieu réactionnel. La réaction est arrêtée après 2 h d'incubation en plaçant les échantillons à 4 °C.

### Analyse HPLC en phase inverse (RP-HPLC)

La procédure est identique à celle indiquée précédemment. Les échantillons sont cependant déposés sur une colonne Jupiter C18 (4,6 mm x 200 mm) (Phenomenex), et les protéines adsorbées sont alors éluées avec un gradient linéaire 0-40 % de phase B.

### Résultats

Les enzymes TET utilisées dans ces expériences sont des métallo-aminopeptidases de la famille M42 (MEROPS). Elles appartiennent toutes à la même famille d'enzyme et possèdent une identité structurale très forte en elles. Par contre, ce sont bien des enzymes différentes avec des spécificités différentes.

Les structures tridimensionnelles des différentes TET utilisées sont très proches alors même qu'elles possèdent toutes des spécificités de substrats différentes. Ainsi PhTET1 est une glutamyl-aminopeptidase, PhTET2 est une leucyl-aminopeptidase avec une activité résiduelle significative envers certains des résidus hydrophobes et polaires non chargé, PhTET3 est une lysyl-aminopeptidase, PhTET4 est une glycyl-aminopeptidase stricte, MjTET est une leucyl-aminopeptidase avec une activité importante envers les résidus hydrophobes et ceux chargés positivement. MjTET est par ailleurs la seule à posséder une activité d'hydrolyse envers les résidus aromatiques.

L'ensemble des résultats présentés ci-dessus ont été obtenus en utilisant des substrats du type monoacyl-pNA (4-nitroaniline) ou monoacyl-AMC (7-amino-4-méthylcoumarine). Ils représentent un peptide de deux résidus, le premier résidu est celui sur lequel est mesurer l'activité de la peptidase, le second est un chromophore qui une fois libéré émet un signal dans le visible. Ainsi, on peut mesurer l'affinité d'une peptidase pour chacun des résidus connus.

Ainsi, plusieurs peptides aux séquences spécifiques ont été conçus et synthétisés (tableau 1 ci-après). D'une part, 5 peptides de 15 résidus dit « enrichis », c'est-à-dire que l'extrémité N-terminale de ces peptides a été enrichi en un type de résidu en particulier :
- Peptide 1 : enrichi en résidus chargés négativement
- Peptide 2 : enrichi en résidus hydrophobes
- Peptide 3 : enrichi en résidus chargés positivement
- Peptide 4 : enrichi en glycines
- Peptide 5 : enrichi en résidus aromatiques

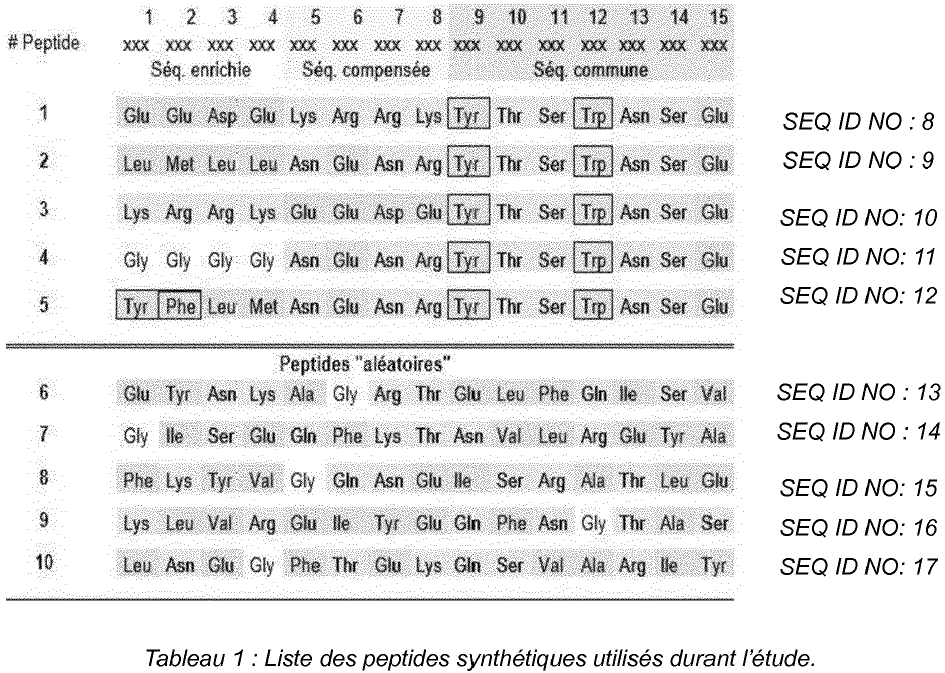

Chacun de ces peptides porte une extrémité N-ter enrichie. Suit alors 4 résidus qui compensent l'effet de la zone enrichie (notamment pour la solubilité du peptide). L'extrémité C-terminale quant à elle est conservée et porte la même série de 7 résidus choisis pour leur absorbance à 280 nm et leur solubilité : YTSWNSE (SEQ ID NO : 7).

D'autre part, 5 peptides dits « aléatoires » c'est-à-dire qu'ils comportent 15 résidus identiques mais répartis selon des séquences différentes : Peptide 6 ; Peptide 7 ; Peptide 8 ; Peptide 9 ; Peptide 10.

Lors des différentes expériences réalisées avec les aminopeptidases TET, en utilisant les peptides ci-dessus comme substrats, plusieurs activités inattendues ont été observées par les inventeurs.

L'aminopeptidase PhTET3 présente une activité optimale contre les résidus chargés positivement, elle est classée en tant que lysyl-aminopeptidase possédant une activité résiduelle contre la leucine, la méthionine, la glutamine et l'aspartate. Pourtant, lors des tests réalisés sur les peptides synthétiques, une activité importante de PhTET3 a été observée sur le peptide enrichi en résidus hydrophobes, le peptide 2 (**figure** 1A).

Le même test a été réalisé avec l'aminopeptidase PhTET2 (**figure** 1B), qui, selon l'état de la technique, est la plus efficace des aminopeptidases PhTET (c'est-à-dire des aminopeptidases provenant des *Thermococcales*) contre les résidus hydrophobes.

Il est alors remarquable de noter que PhTET3 présente une activité plus importante sur le peptide enrichi en résidus hydrophobes que PhTET2 (**figure** 1C). En effet, pendant les 15 minutes du test d'activité, les premiers résidus du peptide ont été hydrolysés plus rapidement en présence de PhTET3 qu'en présence de PhTET2.

Ce type d'activité « inattendue » est également observé dans le cas des peptides 1 et 4 respectivement enrichis en résidus acides glutamiques et glycines. Lorsque ceux-ci sont incubés en présence des peptidases qui, en théorie, présentent le maximum d'activité d'hydrolyse contre les résidus dont leurs extrémités sont enrichies, aucune activité d'hydrolyse n'est observée (**figures** 2A et 2B).

Un autre résultat inattendu a été observé lorsque l'activité de PhTET3 a été mesurée sur un peptide dont l'extrémité N-terminale est enrichie en résidus aromatiques (**Figure** 3A). Il est tout à fait surprenant, compte tenu des données disponibles dans la littérature, d'observer l'hydrolyse de résidus aromatiques par PhTET3. En effet, l'analyse des différents pics de protéines éluées par spectrométrie de masse montre une accumulation importante de peptides dont la tyrosine à l'extrémité N-terminale a été hydrolysée.

Jusqu'à aujourd'hui, la caractérisation des spécificités enzymatiques des aminopeptidases TET a été obtenue en analysant leur activité contre des dipeptides, ce sont les données disponibles dans la littérature. Les tests de l'activité de PhTET3 sur les peptides synthétiques 2 et 5 montrent qu'en réalité, l'activité des aminopeptidases TET peut-être dépendante de la nature du peptide substrat. En effet, alors même que PhTET3 n'est pas capable d'hydrolyser un résidus tyrosine porté par un dipeptide Tyr-pNA, cette dernière montre une grande efficacité d'hydrolyse contre ce résidu dans le contexte du peptide 5. Il n'a pour l'instant pas été montré d'autres activités inattendues du même type, cependant, il n'est pas exclu que des tests à plus grande échelles, i.e. avec une plus grande variété de substrat, ne laissent apparaître d'autres activités.

Les résultats des **figures** 1A, 1B, 1C, 2A et 2B montrent que, malgré le fait que certaines de ces enzymes aient été décrites, leur intégration dans des compositions enzymatiques n'est pas triviale. En effet, en plus des spécificités théoriques de ces aminopeptidases, la nature du peptide alimentaire à modifier et des résidus environnant le site à modifier, en plus des paramètres physico-chimiques du milieu réactionnel, sont à prendre en compte pour concevoir des compositions enzymatiques spécifiques. Ces conclusions, ainsi que les avantages à utiliser des compositions selon l'invention sont mis en évidences dans les expériences qui suivent.

### Hydrolyse accélérée et modulation

### MjTET 90 % / TET3 10 %

Dans le but de démontrer l'intérêt des compositions d'aminopeptidases TET pour l'hydrolyse améliorée de peptides, l'activité d'une composition de 10 % de PhTET3 et de 90 % de MjTET a été testée contre le peptide 5, riche en résidus aromatiques (**figure** 3A-3D). Chaque aminopeptidase seule a été incubée avec le peptide substrat, le milieu réactionnel analysé par RP-HPLC montre que les deux aminopeptidases sont actives sur le peptide. Dans le milieu réactionnel, sont identifiés 4 produits de dégradation, correspondant au peptide substrat à partir duquel a été hydrolysé un (pep-1), deux (pep-2), trois (pep-3) ou quatre résidus (pep-4), en plus du substrat intact (pep-0).

Dans le cas de PhTET3 (**figure** 3A), on note une accumulation du peptide pep-1 alors que le peptide substrat intact n'est, quant à lui, plus présent dans le milieu.

Dans le cas de la peptidase MjTET (**figure** 3B), une partie du peptide intact est encore présente dans le milieu réactionnel, les restes des produits d'hydrolyse sont présents en quantité quasi équivalente. On note une faible accumulation du produit d'hydrolyse final, le peptide pep-4.

Après incubation du peptide en présence de la composition des deux aminopeptidases, le peptide substrat n'est plus présent dans le milieu et on note une accumulation plus importante du peptide pep-4, signifiant que l'hydrolyse du peptide 5 a été plus efficace en présence de la composition comprenant les aminopeptidases TET que lorsque les aminopeptidases sont utilisées seules (**figure** 3C). Les chromatogrammes résultants des trois tests sont superposés sur la **figure** 3D, on peut ainsi observer clairement que l'ajout au mélange de 10 % de l'aminopeptidase PhTET3 (seulement) a permis d'accélérer de façon significative l'hydrolyse d'un peptide, alors même que celle-ci n'a pas une activité optimale sur le peptide.

### MjTET 95% / TET3 5%

La même expérience a été réalisée en modifiant le ratio du mélange TET3 / MjTET, cette fois le peptide a été incubé en présence d'une composition de 95 % de MjTET et 5 % de PhTET3.

Encore une fois, dans le cas de PhTET3, on note une forte accumulation du peptide pep-1 après les 15 minutes d'incubation de l'aminopeptidase avec le peptide substrat (**figure** 4A). Le peptide intact pep-0 est quant à lui encore présent dans le milieu réactionnel en quantité conséquente. Le profil d'hydrolyse du peptide 5 par MjTET présent à 95 % (**figure** 4B) est proche de celui obtenu à 90 % (**figure** 3B).

Ces données, comparées à celles obtenues avec la composition à 95 % de MjTET et 5 % de PhTET3 (**Figure** 4C), montrent encore une fois une accélération de l'hydrolyse du peptide en rajoutant l'aminopeptidase PhTET3 dans le mélange réactionnel **(****Figure** 4D). Dans cas les où les enzymes sont utilisées seules, on observe que les produits de réaction intermédiaires, pep-1, pep-2 et pep-3, sont accumulés. Dans cette dernière expérience, on note une diminution de la concentration de ces intermédiaires, alors qu'il y a une augmentation de celle du produit final pep-4. L'ajout de 5 % de la peptidase PhTET3 permet donc bien d'accélérer le processus d'hydrolyse du peptide.

Il d'autant plus intéressant de noter que dans ce cas précis, l'ajout d'une aminopeptidase qui n'est, en théorie, pas spécifiques des résidus que l'on cherche à hydrolyser permet d'augmenter l'efficacité générale de la composition d'aminopeptidases TET.

Enfin, ces deux exemples de compositions d'aminopeptidases TET, avec modification du ratio, montrent la possibilité qu'offrent les compositions selon l'invention en termes de modulation d'hydrolyse, permettant le cas échant de modifier à façon les peptides substrats plutôt que de les détruire complètement.

Les expériences présentées ci-dessus ont été réalisées à 60 °C. Elles ont également été menées à 40 °C et les données obtenues alors ont été comparées aux précédentes (figure 6).

Lorsque l'aminopeptidase PhTET3 est incubée seule avec le peptide substrat à 40 °C, on observe une diminution importante de l'hydrolyse. En effet, dans ce cas, seul le peptide pep-1 est visible et en très petite quantité (**figure** 5A).

Le même effet est observé dans le cas de MjTET, par contre, tous les peptides intermédiaires sont visibles ainsi que le produit final pep-4, on note uniquement un ralentissement important de l'hydrolyse (**figure** 5B).

Dans le cas d'une composition MjTET à 95 % et PhTET3 à 5% (**figure** 5C), la concentration du produit final pep-4 est largement diminuée. Par contre, il est intéressant de noter que les différents intermédiaires pep-1, pep-2 et pep-3 sont, quant eux, présents en quantité quasi équivalente à l'hydrolyse à 60 °C. Ainsi, l'hydrolyse sur le premier résidu, à savoir la tyrosine, est aussi efficace à 40 °C qu'à 60 °C lorsque la composition de TET est utilisée, alors que celle-ci est fortement diminuée lorsque les aminopeptidases sont utilisées seules. Par contre, la suite du processus d'hydrolyse semble être ralentie, on arrive ainsi au résultat observé où le peptide substrat est encore présent en grande quantité, le produit final, au contraire, est très peu accumulé.

Cet exemple démontre encore une fois la modulation qu'il est possible d'intégrer à la méthode de modification du contenu en polypeptides d'un substrat selon l'invention. Dans cette partie ont été présentées des modulations de quantités (différents ratios d'aminopeptidases TET) et de températures. Il est également possible d'intégrer des modulations de pH et de temps, afin d'obtenir des modifications des peptides plus fines et précises. Ces exemples ont été réalisés avec deux aminopeptidases TET. Toutefois, l'ajout d'autres aminopeptidases TET permet de cibler plus largement, ou plus précisément, des peptides d'intérêts.

### Compositions MjTET/ PhTET4 sur peptide aléatoire

La cinétique de l'hydrolyse du peptide aléatoire 7 par une composition des aminopeptidases MjTET et PhTET4 a été mesurée (**figure** 6). L'hydrolyse du peptide a été analysée par RP-HPLC après 5 min, 15 min et 30 min. Le peptide 7 a la particularité de présenter une glycine à l'extrémité N terminale.

La caractérisation de l'aminopeptidase PhTET4 a montré que c'est une glycine-aminopeptidase. C'est pourquoi, lorsque son activité a été testée sur le peptide 7, seule l'hydrolyse du premier résidu glycine a été observée (**figure** 6A). On peut ainsi voir l'accumulation du peptide pep-1.

Dans le cas de l'hydrolyse du peptide par l'aminopeptidase MjTET, on observe en plus du peptide pep-1, les peptides pep-2 et pep-3 et ce qui correspond au pep-4 (**figure** 6B). On constate par contre, qu'entre 15 et 30 min, la concentration du peptide substrat pep-0 ne semble pas avoir diminué. Dans le même temps, on remarque que la concentration de pep-2 a diminué et que celle de pep-3 a augmenté. Le phénomène que l'on observe ici est dû au fait que l'affinité de l'aminopeptidase pour le produit d'hydrolyse est plus grande que celle du substrat de départ.

Lorsque l'on incube une composition, comprenant les deux aminopeptidase PhTET4 et MjTET dans des proportions équimolaires, mis en contact avec le peptide substrat, on observe une amélioration significative de l'hydrolyse du peptide (**figure** 6C). La concentration du peptide intact pep-0 diminue progressivement entre 5 et 30 min. Celle du peptide pep-1 par contre, augmente dans un premier temps entre 5 et 15 min avant de diminuer fortement entre 15 et 30 min.

Les chromatogrammes obtenus après 30 min d'incubation avec MjTET seule ou avec la composition MjTET et PhTET4 sont superposés sur la **figure** 7. On observe ainsi que les peptides finaux sont présents dans des quantités relativement similaires, alors que le peptide substrat a été quasi totalement hydrolysé. On note que, seule, aucune des deux peptidases n'a été capable d'hydrolyser complètement le premier résidu du peptide durant l'expérience. Des essais avec une troisième peptidase, PhTET3, ont été réalisées.

Dans cet exemple, l'hydrolyse du premier résidu du peptide substrat est accélérée par l'ajout dans la composition d'une peptidase qui, encore une fois, ne présentait pas une activité optimale pour celui-ci. On note qu'en modulant de façon spécifique le type d'aminopeptidase TET, leur ratio, la température ou encore le pH, la méthode de modification permet d'enrichir un mélange de peptide en un des intermédiaires d'hydrolyses observés (dans ce cas le peptide pep-2 enrichis à 15 min sur la **figure** 6B).

### Modulation de l'hydrolyse de peptides au sein d'un mélange complexe

### Hydrolysats de protéines du petit lait

Le petit lait représente la fraction liquide obtenue après la coagulation du lait. Il contient environ 10 % de protéine qui se répartissent en 5 familles principales : la β-lactoglobuline (50 %), l'a-lactalbumine (20 %), les immunoglobulines (10 %), l'albumine de sérum bovin (10 %) et la lactoferrine (2,8 %).

Le substrat utilisé dans les tests qui suivent est un hydrolysat de protéines de petit lait dont la préparation est explicitée plus haut. Afin d'analyser la composition relative en peptide de l'hydrolysat, celui-ci est analysé par chromatographie en phase inverse sur un système HPLC. Le chromatogramme résultant de l'analyse de l'hydrolysat contrôle est présenté sur la figure 9.

Deux hydrolysats ont été préparés à des pH différents afin de pouvoir analyser l'activité des enzymes TET sur ces peptides dans leurs conditions optimales d'activité. Les deux chromatogrammes sont superposés sur la **figure** 9.

On observe ainsi que l'allure générale du chromatogramme reste inchangée. Il n'y a donc pas eu de modification drastique de la composition en peptide lors du changement de pH. Il existe cependant de fines modifications dans le profil d'élution. Ces modifications ponctuelles ont été prises en compte dans l'analyse des résultats.

### Compositions PhTET2/PhTET3 (50/50 %, 90/10 % et 10/90 %)

Ici, il est démontré la possibilité de moduler l'hydrolyse de peptides spécifiques au sein d'un mélange complexe lorsque les aminopeptidases TET sont utilisées en compositions caractéristiques comme celles de l'invention.

Les activités de PhTET2 et PhTET3 sont d'abord mesurées lorsque les aminopeptidases sont utilisées seules **(****Figure** 10). On observe que tous les peptides ne sont pas pris en charge par les aminopeptidases TET, ceci étant dû au fait que les deux aminopeptidases TET utilisées ont des spécificités de substrats différentes et marquées. Le mélange de peptides utilisé pour cette expérience étant un mélange complexe, on observe également des différences dans le degré d'hydrolyse des différents peptides pris en charges par les aminopeptidases. Une partie seulement du chromatogramme est représentée pour plus de clarté, les résultats présentés sont cependant observables sur l'ensemble du chromatogramme.

Lorsque l'on incube l'hydrolysat avec une composition de 2 aminopeptidases en quantités équimolaires, on observe alors une amélioration de l'activité d'hydrolyse sur la plupart des peptides en solutions. De manière inattendue, le degré d'hydrolyse évolue en modifiant le ratio des différentes TET dans la composition (**figure** 11).

Sur la figure 12 sont représentés les chromatogrammes obtenus avec 3 différents ratios de compositions de PhTET2 et de PhTET3. D'abord, le mélange « équimolaire » correspondant à 50 % de chacune des TET, il est également visible sur la **figure** 10. Les chromatogrammes représentés en pointillés correspondent quant à eux à des ratios différents de chaque aminopeptidase TET, 90% de l'une et 10 % de l'autre et vice et versa. On remarque ainsi que les profils d'hydrolyse sont modifiés, signifiant qu'une variation des proportions de chaque aminopeptidase TET dans le mélange modifie le degré d'hydrolyse des peptides en solutions. Une superposition de l'ensemble des différents chromatogrammes est présentée en figure 12 et permet de visualiser de façon claire la modulation possible dans l'hydrolyse des peptides.

Ceci est un résultat surprenant, dans la mesure où il était impossible de le prévoir. Par ailleurs, on remarque que la modulation de l'hydrolyse est différente en fonction des peptides, ainsi, il est possible en modifiant finement les proportions de chaque aminopeptidase TET de la composition de moduler l'hydrolyse des différents peptides. Aucune information dans l'état de la technique ne pouvait laisser penser qu'il était possible de moduler l'hydrolyse de peptides de façon ciblée en variant la concentration des différentes TET dans un mélange.

### Compositions MjTET/ PhTET4 (50/50 %)

Une seconde série de test présentée ci-après concernent les aminopeptidases MjTET et PhTET4. Une superposition d'une fraction des chromatogrammes obtenus est présentée sur la **figure** 13. Dans ce cas on observe que l'aminopeptidase PhTET4, spécifique des résidus glycines, n'est pas capable d'hydrolyser des peptides lorsqu'elle est utilisée seule. On remarque ainsi une très nette superposition du chromatogramme contrôle et celui obtenu après incubation avec PhTET4. Au contraire, MjTET est, quant à elle, capable de prendre en charge et d'hydrolyser plusieurs peptides présents dans le mélange de peptide.

Le résultat remarquable dans cette série de test est lié au fait que, lorsqu'on mélange MjTET à PhTET4 dans une composition, on observe une augmentation significative de l'hydrolyse des peptides du substrat.

Encore une fois, ce résultat non prévisible montre combien il est possible de moduler l'hydrolyse de différents peptides de façon spécifique dans un mélange en utilisant une composition d'aminopeptidases TET caractéristique. Les différents résultats obtenus jusqu'à aujourd'hui montrent qu'il est également possible de moduler l'activité des aminopeptidases TET en fonction des conditions physico-chimique du milieu réactionnel. Nous proposons ainsi un procédé basé sur les propriétés exceptionnelles de ces enzymes.

### Composition Ph TET1/Ph TET2/Ph TET3/Thermolysine

Le petit lait utilisé dans ces expériences provient d'une fromagerie de Haute-Savoie. Le petit lait a été transporté à 4 °C avant d'être fractionné en échantillons de 1 mL conservés à -20 °C.

Après incubation avec la thermolysine (**figure** 14), on observe un grand nombre de petits peptides présents dans l'échantillons, résultat de l'hydrolyse des protéines du petit lait par la thermolysine.

Il est remarquable de noter qu'après incubation avec la thermolysine et les TET, la grande majorité de ces peptides a été dégradée. On note l'enrichissement de quelques peptides qui représentent les produits de dégradation liés à l'activité des aminopeptidases TET.

### Hydrolyse d'un peptide spécifique au sein d'un d'hydrolysat de caséine - composition PhTET3, MjTET et PhTET4 (33/33/33 %)

Dans cette expérience, le peptide synthétique 7 a été incorporé à un mélange de peptide complexe, un hydrolysat de caséine (Sigma). Après incubation avec une composition d'aminopeptidases PhTET3, MjTET et PhTET4, le milieu réactionnel a été analysé par RP-HPLC. Les résultats des différentes expériences menées sont présentés en **figure** 15. Le peptide peut être identifié sans ambiguïté et ses premières étapes de dégradation on put être observées. La complexité du milieu a rendu l'analyse des fragments courts plus complexe.

Lorsque le mélange de l'hydrolysat de caséine et du peptide 7 est incubé avec les aminopeptidases PhTET3 ou MjTET, on observe que le peptide d'intérêt est peu hydrolysé (**figure** 15A et B). Comme on peut s'y attendre par contre, un faible nombre de peptides issus de l'hydrolyse de la caséine du mélange sont au moins en partie hydrolysé par les aminopeptidases TET.

Lorsque le mélange est incubé en présence de PhTET4, seul le peptide d'intérêt est hydrolysé par l'aminopeptidase PhTET4 et ce, de façon quasi-totale (**figure** 15C), le seul peptide apparu étant le peptide pep-1.

Le peptide d'intérêt intact pep-0 est également totalement absent lorsque le mélange de peptides est incubé en présence de la composition des trois aminopeptidases TET (**figure** 15D). Par contre, dans ce dernier cas, on peut remarquer que le peptide pep-1 a lui-même été hydrolysé puisque sa concentration a diminué de façon significative par rapport à l'expérience avec PhTET4 seule.

Cette expérience montre que la méthode permet de cibler de façon précise un peptide dans un mélange afin de le modifier ou de l'éliminer.

### Hydrolyse spécifique d'une partie des protéines natives du gluten

### Composition PhTET1/PhTET2/PhTET3 (33/33/33 %)

Le gluten est un mélange de différentes protéines classées en 2 grandes familles, les gluténine et les gliadines. Certaines gliadines sont porteuses d'un peptide dit « immunodominant » qui provoque une réaction allergique chez les personnes sensibles ou intolérantes au gluten, ce syndrome est plus connu sous le nom de la maladie de Coeliaque.

Sur les chromatogrammes présentés en **figure** 16, on observe qu'après incubation d'un échantillon de gluten total solubilisé dans du propanol avec une composition des aminopeptidases PhTET1, PhTET2 et PhTET3 en quantité équimolaire, on note la diminution significative de la concentration en gliadine dans l'échantillon.

Dans ce cas, la composition des aminopeptidases PhTET1, PhTET2 et PhTET3, seules, c'est à dire sans ajout d'endopeptidase, ont permis de diminuer la concentration des protéines porteuse du peptide immunodominant dans un échantillon de gluten total solubilisé dans une solution à 50 % de propanol.

### Composition PhTET1/PhTET2/PhTET3/thermolysine

Après incubation du gluten avec l'endoprotéase thermolysine, on note en **figure** 17 un nombre important de petit peptide dans l'échantillon résultant de l'hydrolyse très efficace des protéines du gluten par l'endoprotéase. Lorsque les aminopeptidases PhTET1, PhTET2 et PhTET3 sont intégrées à la composition, on note une diminution de la grande majorité des pics d'absorbance, ce qui traduit une hydrolyse de l'ensemble de ces pics par les aminopeptidases.

On note également un enrichissement de quelques pics qui représentent les produits de dégradation des aminopeptidases.

## Revendications

1. Composition comprenant au moins une première aminopeptidase et au moins une deuxième aminopeptidase, lesdites première et deuxième aminopeptidases différentes l'une de l'autre, lesdites première et deuxième aminopeptidases étant issues de microorganismes extrêmophiles, lesdites première et deuxième aminopeptidases étant des aminopeptidases de la famille des aminopeptidases tétraédriques ou aminopeptidases TET, ladite première aminopeptidase représentant jusqu'à 40 % en poids par rapport au poids total de la composition, et si lesdites première et deuxième aminopeptidases sont différentes de PhTET2 et PhTET3, alors ladite première aminopeptidase représente jusqu'à 50 % en poids par rapport au poids total de la composition.

2. Composition selon la revendication 1, dans laquelle ladite au moins une première aminopeptidase et ladite au moins une deuxième aminopeptidase sont choisies parmi les aminopeptidases du groupe constitué de : PhTET1, PhTET2, PhTET3, PhTET4 et MjTET.

3. Composition selon la revendication 2, dans laquelle lesdites aminopeptidases comprennent, consistent essentiellement ou consistent en les molécules d'acides aminés de séquences respectives SEQ ID NO : 1 à SEQ ID NO : 5, ou des protéines présentant une activité aminopeptidase, lesdites protéines comprenant, consistant essentiellement ou consistant en des molécules d'acides aminés dont les séquences présentent au moins 65 % d'identité avec l'une des séquences SEQ ID NO : 1 à SEQ ID NO : 5.

4. Composition selon l'une quelconque des revendications précédentes, dans laquelle ladite première aminopeptidase représente jusqu'à 10 % en poids par rapport au poids total de la composition, notamment jusqu'à 5 % en poids par rapport au poids total de la composition.

5. Composition selon l'une quelconque des revendications 1 à 4, dans laquelle ladite première aminopeptidase représente 50 % en poids par rapport au poids total de la composition, sous réserve que lesdites première et deuxième aminopeptidases sont différentes de PhTET2 et PhTET3.

6. Composition selon l'une quelconque des revendications 1 à 4, comprenant au moins une troisième aminopeptidase, ladite troisième aminopeptidase étant une aminopeptidase de la famille des aminopeptidases tétraédriques ou aminopeptidases TET.

7. Composition selon la revendication 6, dans laquelle lesdites première, deuxième et troisième aminopeptidases sont dans des proportions équimolaires ou sensiblement équimolaires.

8. Composition selon l'une quelconque des revendications précédentes, comprenant en outre une endopeptidase, notamment la thermolysine, en particulier la thermolysine de séquence SEQ ID NO : 6.

9. Utilisation d'une composition selon l'une quelconque des revendications précédentes pour la modification de tout ou partie du contenu en polypeptides d'un substrat comprenant des peptides, des polypeptides et/ou des protéines.

10. Utilisation selon la revendication 9, dans laquelle le substrat comprend au moins des peptides, polypeptides et/ou des protéines du gluten et/ou du lactosérum.

11. Utilisation selon la revendication 9 ou 10, dans laquelle le substrat comprend au moins une des protéines suivantes : gliadine, β-lactoglobuline, l'a-lactalbumine, immunoglobulines, albumine de sérum et lactoferrine.

12. Utilisation selon l'une quelconque des revendications 9 à 11, dans laquelle lesdites aminopeptidases sont utilisées de manière simultanée, séparée ou étalée dans le temps.

13. Méthode de modification de tout ou partie du contenu en polypeptides d'un substrat comprenant des peptides, polypeptides et/ou des protéines, ladite méthode comprenant une étape de mise en contact :
• dudit substrat avec
• une composition selon l'une quelconque des revendications 1 à 8,
ladite au moins une première aminopeptidase et ladite au moins deuxième aminopeptidase pouvant être activées à une température supérieure à 80°C, et
comprenant éventuellement, préalablement à ladite étape de mise en contact, une étape de dénaturation des polypeptides dudit substrat.

14. Composé alimentaire susceptible d'être obtenu par la méthode selon la revendication 13.

15. Composé alimentaire comprenant au moins une des protéines suivantes sous forme modifiée : gliadine, β-lactoglobuline, l'a-lactalbumine, immunoglobulines, albumine de sérum et lactoferrine, ledit composé alimentaire comprenant en outre une composition selon l'une quelconque des revendications 1 à 8.


SEQUENCE LISTING

<110>  CENTRE NATIONAL DE LA RECHERCHE SCIENTIFIQUE

<120>  Utilisation d'enzymes pour modifier des polypeptides

<130>  BR82390

<160>  17

<170>  PatentIn version 3.5

<210>  1
<211>  332
<212>  PRT
<213>  Pyrococcus horikoshii

<400>  1

Met Met Ser Met Ile Glu Lys Leu Lys Lys Phe Thr Gln Ile Pro Gly
1               5                   10                  15

Ile Ser Gly Tyr Glu Glu Arg Ile Arg Glu Glu Ile Ile Arg Glu Ile
            20                  25                  30

Lys Asp Phe Ala Asp Tyr Lys Val Asp Ala Ile Gly Asn Leu Ile Val
        35                  40                  45

Glu Leu Gly Glu Gly Glu Glu Arg Ile Leu Phe Met Ala His Met Asp
    50                  55                  60

Glu Ile Gly Leu Leu Ile Thr Gly Ile Thr Asp Glu Gly Lys Leu Arg
65                  70                  75                  80

Phe Arg Lys Val Gly Gly Ile Asp Asp Arg Leu Leu Tyr Gly Arg His
                85                  90                  95

Val Asn Val Val Thr Glu Lys Gly Ile Leu Asp Gly Val Ile Gly Ala
            100                 105                 110

Thr Pro Pro His Leu Ser Leu Glu Arg Asp Lys Ser Val Ile Pro Trp
        115                 120                 125

Tyr Asp Leu Val Ile Asp Ile Gly Ala Glu Ser Lys Glu Glu Ala Leu
    130                 135                 140

Glu Leu Val Lys Pro Leu Asp Phe Ala Val Phe Lys Lys His Phe Ser
145                 150                 155                 160

Val Leu Asn Gly Lys Tyr Val Ser Thr Arg Gly Leu Asp Asp Arg Phe
                165                 170                 175

Gly Val Val Ala Leu Ile Glu Ala Ile Lys Asp Leu Val Asp His Glu
            180                 185                 190

Leu Glu Gly Lys Val Ile Phe Ala Phe Thr Val Gln Glu Glu Val Gly
        195                 200                 205

Leu Lys Gly Ala Lys Phe Leu Ala Asn His Tyr Tyr Pro Gln Tyr Ala
    210                 215                 220

Phe Ala Ile Asp Ser Phe Ala Cys Cys Ser Pro Leu Thr Gly Asp Val
225                 230                 235                 240

Lys Leu Gly Lys Gly Pro Val Ile Arg Ala Val Asp Asn Ser Ala Ile
                245                 250                 255

Tyr Ser Arg Asp Leu Ala Arg Lys Val Trp Ser Ile Ala Glu Lys Asn
            260                 265                 270

Gly Ile Glu Ile Gln Ile Gly Val Thr Gly Gly Gly Thr Asp Ala Ser
        275                 280                 285

Ala Phe Gln Asp Arg Ser Lys Thr Leu Ala Leu Ser Val Pro Ile Lys
    290                 295                 300

Tyr Leu His Ser Glu Val Glu Thr Leu His Leu Asn Asp Leu Glu Lys
305                 310                 315                 320

Leu Val Lys Leu Ile Glu Ala Leu Ala Phe Glu Leu
                325                 330

<210>  2
<211>  353
<212>  PRT
<213>  PYROCOCCUS HORIKOSHII

<400>  2

Met Glu Val Arg Asn Met Val Asp Tyr Glu Leu Leu Lys Lys Val Val
1               5                   10                  15

Glu Ala Pro Gly Val Ser Gly Tyr Glu Phe Leu Gly Ile Arg Asp Val
            20                  25                  30

Val Ile Glu Glu Ile Lys Asp Tyr Val Asp Glu Val Lys Val Asp Lys
        35                  40                  45

Leu Gly Asn Val Ile Ala His Lys Lys Gly Glu Gly Pro Lys Val Met
    50                  55                  60

Ile Ala Ala His Met Asp Gln Ile Gly Leu Met Val Thr His Ile Glu
65                  70                  75                  80

Lys Asn Gly Phe Leu Arg Val Ala Pro Ile Gly Gly Val Asp Pro Lys
                85                  90                  95

Thr Leu Ile Ala Gln Arg Phe Lys Val Trp Ile Asp Lys Gly Lys Phe
            100                 105                 110

Ile Tyr Gly Val Gly Ala Ser Val Pro Pro His Ile Gln Lys Pro Glu
        115                 120                 125

Asp Arg Lys Lys Ala Pro Asp Trp Asp Gln Ile Phe Ile Asp Ile Gly
    130                 135                 140

Ala Glu Ser Lys Glu Glu Ala Glu Asp Met Gly Val Lys Ile Gly Thr
145                 150                 155                 160

Val Ile Thr Trp Asp Gly Arg Leu Glu Arg Leu Gly Lys His Arg Phe
                165                 170                 175

Val Ser Ile Ala Phe Asp Asp Arg Ile Ala Val Tyr Thr Ile Leu Glu
            180                 185                 190

Val Ala Lys Gln Leu Lys Asp Ala Lys Ala Asp Val Tyr Phe Val Ala
        195                 200                 205

Thr Val Gln Glu Glu Val Gly Leu Arg Gly Ala Arg Thr Ser Ala Phe
    210                 215                 220

Gly Ile Glu Pro Asp Tyr Gly Phe Ala Ile Asp Val Thr Ile Ala Ala
225                 230                 235                 240

Asp Ile Pro Gly Thr Pro Glu His Lys Gln Val Thr His Leu Gly Lys
                245                 250                 255

Gly Thr Ala Ile Lys Ile Met Asp Arg Ser Val Ile Cys His Pro Thr
            260                 265                 270

Ile Val Arg Trp Leu Glu Glu Leu Ala Lys Lys His Glu Ile Pro Tyr
        275                 280                 285

Gln Leu Glu Ile Leu Leu Gly Gly Gly Thr Asp Ala Gly Ala Ile His
    290                 295                 300

Leu Thr Lys Ala Gly Val Pro Thr Gly Ala Leu Ser Val Pro Ala Arg
305                 310                 315                 320

Tyr Ile His Ser Asn Thr Glu Val Val Asp Glu Arg Asp Val Asp Ala
                325                 330                 335

Thr Val Glu Leu Met Thr Lys Ala Leu Glu Asn Ile His Glu Leu Lys
            340                 345                 350

Ile

<210>  3
<211>  354
<212>  PRT
<213>  PYROCOCCUS HORIKOSHII

<400>  3

Met Asp Leu Lys Gly Gly Glu Ser Met Val Asp Trp Lys Leu Met Gln
1               5                   10                  15

Glu Ile Ile Glu Ala Pro Gly Val Ser Gly Tyr Glu His Leu Gly Ile
            20                  25                  30

Arg Asp Ile Val Val Asp Val Leu Lys Glu Val Ala Asp Glu Val Lys
        35                  40                  45

Val Asp Lys Leu Gly Asn Val Ile Ala His Phe Lys Gly Ser Ser Pro
    50                  55                  60

Arg Ile Met Val Ala Ala His Met Asp Lys Ile Gly Val Met Val Asn
65                  70                  75                  80

His Ile Asp Lys Asp Gly Tyr Leu His Ile Val Pro Ile Gly Gly Val
                85                  90                  95

Leu Pro Glu Thr Leu Val Ala Gln Arg Ile Arg Phe Phe Thr Glu Lys
            100                 105                 110

Gly Glu Arg Tyr Gly Val Val Gly Val Leu Pro Pro His Leu Arg Arg
        115                 120                 125

Gly Gln Glu Asp Lys Gly Ser Lys Ile Asp Trp Asp Gln Ile Val Val
    130                 135                 140

Asp Val Gly Ala Ser Ser Lys Glu Glu Ala Glu Glu Met Gly Phe Arg
145                 150                 155                 160

Val Gly Thr Val Gly Glu Phe Ala Pro Asn Phe Thr Arg Leu Asn Glu
                165                 170                 175

His Arg Phe Ala Thr Pro Tyr Leu Asp Asp Arg Ile Cys Leu Tyr Ala
            180                 185                 190

Met Ile Glu Ala Ala Arg Gln Leu Gly Asp His Glu Ala Asp Ile Tyr
        195                 200                 205

Ile Val Gly Ser Val Gln Glu Glu Val Gly Leu Arg Gly Ala Arg Val
    210                 215                 220

Ala Ser Tyr Ala Ile Asn Pro Glu Val Gly Ile Ala Met Asp Val Thr
225                 230                 235                 240

Phe Ala Lys Gln Pro His Asp Lys Gly Lys Ile Val Pro Glu Leu Gly
                245                 250                 255

Lys Gly Pro Val Met Asp Val Gly Pro Asn Ile Asn Pro Lys Leu Arg
            260                 265                 270

Ala Phe Ala Asp Glu Val Ala Lys Lys Tyr Glu Ile Pro Leu Gln Val
        275                 280                 285

Glu Pro Ser Pro Arg Pro Thr Gly Thr Asp Ala Asn Val Met Gln Ile
    290                 295                 300

Asn Lys Glu Gly Val Ala Thr Ala Val Leu Ser Ile Pro Ile Arg Tyr
305                 310                 315                 320

Met His Ser Gln Val Glu Leu Ala Asp Ala Arg Asp Val Asp Asn Thr
                325                 330                 335

Ile Lys Leu Ala Lys Ala Leu Leu Glu Glu Leu Lys Pro Met Asp Phe
            340                 345                 350

Thr Pro

<210>  4
<211>  336
<212>  PRT
<213>  PYROCOCCUS HORIKOSHII

<400>  4

Met Glu Arg Ile Val Lys Ile Leu Arg Glu Ile Leu Glu Ile Pro Ser
1               5                   10                  15

Pro Thr Gly Tyr Thr Lys Glu Val Met Ser Tyr Leu Glu Lys Phe Leu
            20                  25                  30

Lys Glu Asn Glu Val Asn Phe Tyr Tyr Thr Asn Lys Gly Ala Leu Ile
        35                  40                  45

Ala Gly Asn His Pro Lys Pro Glu Leu Val Val Ile Ala His Val Asp
    50                  55                  60

Thr Leu Gly Ala Met Val Lys Glu Ile Leu Pro Asp Gly His Leu Ala
65                  70                  75                  80

Phe Ser Arg Ile Gly Gly Leu Val Leu Pro Thr Phe Glu Gly Glu Tyr
                85                  90                  95

Cys Thr Ile Ile Thr Arg Lys Gly Lys Lys Phe Arg Gly Thr Leu Leu
            100                 105                 110

Leu Arg Asn Pro Ser Ala His Val Asn Arg Glu Val Gly Lys Lys Glu
        115                 120                 125

Arg Lys Glu Glu Asn Met Tyr Ile Arg Leu Asp Glu Leu Val Glu Lys
    130                 135                 140

Arg Glu Asp Thr Glu Lys Leu Gly Ile Arg Pro Gly Asp Phe Ile Ala
145                 150                 155                 160

Phe Asp Pro Lys Phe Glu Tyr Val Asn Gly Phe Val Lys Ser His Phe
                165                 170                 175

Leu Asp Asp Lys Ala Ser Val Ala Ala Ile Leu Asp Leu Ile Ile Asp
            180                 185                 190

Met Lys Asp Glu Leu Glu Lys Tyr Pro Val Ala Phe Phe Phe Ser Pro
        195                 200                 205

Tyr Glu Glu Val Gly His Gly Gly Ser Ala Gly Tyr Pro Pro Thr Thr
    210                 215                 220

Lys Glu Leu Leu Val Val Asp Met Gly Val Val Gly Glu Gly Val Ser
225                 230                 235                 240

Gly Lys Glu Thr Ala Val Ser Ile Ala Ala Lys Asp Thr Thr Gly Pro
                245                 250                 255

Tyr Asp Tyr Asp Met Thr Asn Arg Leu Ile Glu Leu Ala Glu Glu Asn
            260                 265                 270

Asn Ile Pro Tyr Val Val Asp Val Phe Pro Tyr Tyr Gly Ser Asp Gly
        275                 280                 285

Ser Ala Ala Leu Arg Ala Gly Trp Asp Phe Arg Val Ala Leu Ile Gly
    290                 295                 300

Pro Gly Val His Ala Ser His Gly Met Glu Arg Thr His Val Lys Gly
305                 310                 315                 320

Leu Leu Ala Thr Lys Glu Leu Ile Arg Ala Tyr Ile Lys Trp Lys Gly
                325                 330                 335

<210>  5
<211>  350
<212>  PRT
<213>  METHANOCALDOCOCCUS JANNASCHII

<400>  5

Met Ser Val Val Glu Tyr Leu Lys Lys Leu Ser Lys Leu His Gly Ile
1               5                   10                  15

Ser Gly Arg Glu Asp Ser Val Arg Glu Phe Met Lys Lys Glu Leu Glu
            20                  25                  30

Lys Tyr Cys Asp Ser Val Glu Ile Asp Asn Phe Gly Asn Leu Ile Ala
        35                  40                  45

Lys Arg Gly Asn Lys Gly Lys Lys Ile Met Ile Ala Ala His Met Asp
    50                  55                  60

Glu Ile Gly Leu Met Val Lys Tyr Ile Asp Asp Asn Gly Phe Leu Lys
65                  70                  75                  80

Phe Thr Lys Ile Gly Gly Ile Tyr Asp Pro Thr Ile Leu Asn Gln Lys
                85                  90                  95

Val Val Val His Gly Ser Lys Gly Asp Leu Ile Gly Val Leu Gly Ser
            100                 105                 110

Lys Pro Pro His Arg Met Lys Glu Glu Glu Lys Thr Lys Ile Ile Lys
        115                 120                 125

Tyr Glu Asp Met Phe Ile Asp Ile Gly Ala Glu Ser Arg Glu Glu Ala
    130                 135                 140

Ile Glu Met Gly Val Asn Ile Gly Thr Trp Val Ser Phe Leu Ser Glu
145                 150                 155                 160

Val Tyr Asp Leu Gly Lys Asn Arg Leu Thr Gly Lys Ala Phe Asp Asp
                165                 170                 175

Arg Val Gly Cys Ala Val Leu Leu Glu Val Met Lys Arg Leu Ser Glu
        180                 185                 190

Glu Asp Ile Asp Cys Gln Val Tyr Ala Val Gly Thr Val Gln Glu Glu
        195                 200                 205

Val Gly Leu Lys Gly Ala Arg Val Ser Ala Phe Lys Ile Asn Pro Asp
    210                 215                 220

Val Ala Ile Ala Leu Asp Val Thr Ile Ala Gly Asp His Pro Gly Ile
225                 230                 235                 240

Lys Lys Glu Asp Ala Pro Val Asp Leu Gly Lys Gly Pro Val Val Gly
                245                 250                 255

Ile Val Asp Ala Ser Gly Arg Gly Leu Ile Ala His Pro Lys Val Leu
            260                 265                 270

Asp Met Ile Lys Ala Val Ser Glu Lys Tyr Lys Ile Asp Val Gln Trp
        275                 280                 285

Glu Val Gly Glu Gly Gly Thr Thr Asp Ala Thr Ala Ile His Leu Thr
    290                 295                 300

Arg Glu Gly Ile Pro Thr Gly Val Ile Ser Val Pro Ala Arg Tyr Ile
305                 310                 315                 320

His Thr Pro Val Glu Val Ile Asp Lys Arg Asp Leu Glu Lys Thr Val
                325                 330                 335

Glu Leu Val Tyr Asn Cys Ile Lys Glu Val Asn Asn Phe Phe
            340                 345                 350

<210>  6
<211>  551
<212>  PRT
<213>  GEOBACILLUS STEAROTHERMOPHILUS

<400>  6

Met Lys Arg Lys Met Lys Met Lys Leu Val Arg Phe Gly Leu Ala Ala
1               5                   10                  15

Gly Leu Ala Ala Gln Val Phe Phe Leu Pro Tyr Asn Ala Leu Ala Ser
            20                  25                  30

Thr Glu His Val Thr Trp Asn Gln Gln Phe Gln Thr Pro Gln Phe Ile
        35                  40                  45

Ser Gly Asp Leu Leu Lys Val Asn Gly Thr Ser Pro Glu Glu Leu Val
50                  55                  60

Tyr Gln Tyr Val Glu Lys Asn Glu Asn Lys Phe Lys Phe His Glu Asn
65                  70                  75                  80

Ala Lys Asp Thr Leu Gln Leu Lys Glu Lys Lys Asn Asp Asn Leu Gly
                85                  90                  95

Phe Thr Phe Met Arg Phe Gln Gln Thr Tyr Lys Gly Ile Pro Val Phe
            100                 105                 110

Gly Ala Val Val Thr Ala His Val Lys Asp Gly Thr Leu Thr Ala Leu
        115                 120                 125

Ser Gly Thr Leu Ile Pro Asn Leu Asp Thr Lys Gly Ser Leu Lys Ser
    130                 135                 140

Gly Lys Lys Leu Ser Glu Lys Gln Ala Arg Asp Ile Ala Glu Lys Asp
145                 150                 155                 160

Leu Val Ala Asn Val Thr Lys Glu Val Pro Glu Tyr Glu Gln Gly Lys
                165                 170                 175

Asp Thr Glu Phe Val Val Tyr Val Asn Gly Asp Glu Ala Ser Leu Ala
            180                 185                 190

Tyr Val Val Asn Leu Asn Phe Leu Thr Pro Glu Pro Gly Asn Trp Leu
        195                 200                 205

Tyr Ile Ile Asp Ala Val Asp Gly Lys Ile Leu Asn Lys Phe Asn Gln
    210                 215                 220

Leu Asp Ala Ala Lys Pro Gly Asp Val Lys Ser Ile Thr Gly Thr Ser
225                 230                 235                 240

Thr Val Gly Val Gly Arg Gly Val Leu Gly Asp Gln Lys Asn Ile Asn
                245                 250                 255

Thr Thr Tyr Ser Thr Tyr Tyr Tyr Leu Gln Asp Asn Thr Arg Gly Asn
            260                 265                 270

Gly Ile Phe Thr Tyr Asp Ala Lys Tyr Arg Thr Thr Leu Pro Gly Ser
        275                 280                 285

Leu Trp Ala Asp Ala Asp Asn Gln Phe Phe Ala Ser Tyr Asp Ala Pro

290                 295                 300

Ala Val Asp Ala His Tyr Tyr Ala Gly Val Thr Tyr Asp Tyr Tyr Lys
305                 310                 315                 320

Asn Val His Asn Arg Leu Ser Tyr Asp Gly Asn Asn Ala Ala Ile Arg
                325                 330                 335

Ser Ser Val His Tyr Ser Gln Gly Tyr Asn Asn Ala Phe Trp Asn Gly
            340                 345                 350

Ser Gln Met Val Tyr Gly Asp Gly Asp Gly Gln Thr Phe Ile Pro Leu
        355                 360                 365

Ser Gly Gly Ile Asp Val Val Ala His Glu Leu Thr His Ala Val Thr
    370                 375                 380

Asp Tyr Thr Ala Gly Leu Ile Tyr Gln Asn Glu Ser Gly Ala Ile Asn
385                 390                 395                 400

Glu Ala Ile Ser Asp Ile Phe Gly Thr Leu Val Glu Phe Tyr Ala Asn
                405                 410                 415

Lys Asn Pro Asp Trp Glu Ile Gly Glu Asp Val Tyr Thr Pro Gly Ile
            420                 425                 430

Ser Gly Asp Ser Leu Arg Ser Met Ser Asp Pro Ala Lys Tyr Gly Asp
        435                 440                 445

Pro Asp His Tyr Ser Lys Arg Tyr Thr Gly Thr Gln Asp Asn Gly Gly
    450                 455                 460

Val His Ile Asn Ser Gly Ile Ile Asn Lys Ala Ala Tyr Leu Ile Ser
465                 470                 475                 480

Gln Gly Gly Thr His Tyr Gly Val Ser Val Val Gly Ile Gly Arg Asp
                485                 490                 495

Lys Leu Gly Lys Ile Phe Tyr Arg Ala Leu Thr Gln Tyr Leu Thr Pro
            500                 505                 510

Thr Ser Asn Phe Ser Gln Leu Arg Ala Ala Ala Val Gln Ser Ala Thr
        515                 520                 525

Asp Leu Tyr Gly Ser Thr Ser Gln Glu Val Ala Ser Val Lys Gln Ala
    530                 535                 540

Phe Asp Ala Val Gly Val Lys
545                 550


<210>  7
<211>  7
<212>  PRT
<213>  Artificial sequence

<220>
<223>  Synthetic peptide

<400>  7

Tyr Thr Ser Trp Asn Ser Glu
1               5


<210>  8
<211>  15
<212>  PRT
<213>  Artificial sequence

<220>
<223>  synthetic peptide

<400>  8

Glu Glu Asp Glu Lys Arg Arg Lys Tyr Thr Ser Trp Asn Ser Glu
1               5                   10                  15


<210>  9
<211>  15
<212>  PRT
<213>  Artificial sequence

<220>
<223>  synthetic peptide

<400>  9

Leu Met Leu Leu Asn Glu Asn Arg Tyr Thr Ser Trp Asn Ser Glu
1               5                   10                  15


<210>  10
<211>  15
<212>  PRT
<213>  Artificial sequence

<220>
<223>  synthetic peptide

<400>  10

Lys Arg Arg Lys Glu Glu Asp Glu Tyr Thr Ser Trp Asn Ser Glu
1               5                   10                  15


<210>  11
<211>  15
<212>  PRT

<213>  Artificial sequence

<220>
<223>  synthetic peptide

<400>  11

Gly Gly Gly Gly Asn Glu Asn Arg Tyr Thr Ser Trp Asn Ser Glu
1               5                   10                  15


<210>  12
<211>  15
<212>  PRT
<213>  Artificial sequence

<220>
<223>  synthetic peptide

<400>  12

Tyr Phe Leu Met Asn Glu Asn Arg Tyr Thr Ser Trp Asn Ser Glu
1               5                   10                  15


<210>  13
<211>  15
<212>  PRT
<213>  Artificial sequence

<220>
<223>  synthetic peptide

<400>  13

Glu Tyr Asn Lys Ala Gly Arg Thr Glu Leu Phe Gln Ile Ser Val
1               5                   10                  15


<210>  14
<211>  15
<212>  PRT
<213>  Artificial sequence

<220>
<223>  synthetic peptide

<400>  14

Gly Ile Ser Glu Gln Phe Lys Thr Asn Val Leu Arg Glu Tyr Ala
1               5                   10                  15


<210>  15
<211>  15
<212>  PRT
<213>  Artificial sequence

<220>
<223>  synthetic peptide

<400>  15

Phe Lys Tyr Val Gly Gln Asn Glu Ile Ser Arg Ala Thr Leu Glu
1               5                   10                  15

<210>  16
<211>  15
<212>  PRT
<213>  Artificial sequence

<220>
<223>  synthetic peptide

<400>  16

Lys Leu Val Arg Glu Ile Tyr Glu Gln Phe Asn Gly Thr Ala Ser
1               5                   10                  15

<210>  17
<211>  15
<212>  PRT
<213>  Artificial sequence

<220>
<223>  synthetic peptide

<400>  17

Leu Asn Glu Gly Phe Thr Glu Lys Gln Ser Val Ala Arg Ile Tyr
1               5                   10                  15